# EUROPEAN PATENT APPLICATION

(11) **EP 3 858 372 A1**
(43) Date of publication of application: **04.08.2021**
(21) Application number: 20000053.7
(22) Date of filing: 31.01.2020
(51) Int. Cl.: A61K 38/21, A61K 38/22, A61M 11/00, A61M 15/00

(54) **HUMAN ANTI-INFLAMMATORY PEPTIDES FOR THE INHALATORY TREATMENT OF INFLAMMATORY PULMONARY DISEASES**

(71) Applicant: AdVita Lifescience GmbH, 79211 Denzlingen (DE)
(72) Inventor: BEVEC, Dorian, 61231 Bad Nauheim (DE); VON WEGERER, Jörg, 13597 Berlin (DE)
(74) Representative: Maucher Jenkins Patent- und Rechtsanwälte

(57) **Abstract**

The present invention relates to the use of human anti-inflammatory peptides in the inhalatory treatment of inflammatory pulmonary diseases. The invention in particular relates to the use of vasoactive intestinal peptide, C-type natriuretic peptide, B-type natriuretic peptide, pituitary adenylate cyclase-activating peptide, adrenomedullin, alpha-melanocyte stimulating hormone, relaxin and interferon gamma for said purposes. Advantageous features of an aerosol containing such a human anti-inflammatory peptide and a method for producing said aerosol are disclosed. The present invention further relates to a kit for inhalatory treatment of inflammatory pulmonary diseases.

SEQUENCE LISTING

<110> Bevec Dorian

<120> Human anti-inflammatory peptides for the inhalatory treatment of
inflammatory pulmonary diseases

<130> DB-001/EP-P

<160> 14

<170> BiSSAP 1.3.6

<210> 1
<211> 28
<212> PRT
<213> Artificial Sequence

<220>
<223> Vasoactive Intestinal Peptide

<400> 1
His Ser Asp Ala Val Phe Thr Asp Asn Tyr Thr Arg Leu Arg Lys Gln
1               5                   10                  15
Met Ala Val Lys Lys Tyr Leu Asn Ser Ile Leu Asn
            20                  25

<210> 2
<211> 22
<212> PRT
<213> Artificial Sequence

<220>
<223> C-type Natriuretic Peptide

<400> 2
Gly Leu Ser Lys Gly Cys Phe Gly Leu Lys Leu Asp Arg Ile Gly Ser
1               5                   10                  15
Met Ser Gly Leu Gly Cys
            20

<210> 3
<211> 32
<212> PRT
<213> Artificial Sequence

<220>
<223> B-type Natriuretic Peptide

<400> 3
Ser Pro Lys Met Val Gln Gly Ser Gly Cys Phe Gly Arg Lys Met Asp
1               5                   10                  15
Arg Ile Ser Ser Ser Ser Gly Leu Gly Cys Lys Val Leu Arg Arg His
            20                  25                  30

<210> 4
<211> 27
<212> PRT

<213> Artificial Sequence

<220>
<223> Pituitary Adenylate Cyclase-activating Peptide 27

<400> 4
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu
            20                  25

<210> 5
<211> 38
<212> PRT
<213> Artificial Sequence

<220>
<223> Pituitary Adenylate Cyclase-activating Peptide 38

<400> 5
His Ser Asp Gly Ile Phe Thr Asp Ser Tyr Ser Arg Tyr Arg Lys Gln
1               5                   10                  15
Met Ala Val Lys Lys Tyr Leu Ala Ala Val Leu Gly Lys Arg Tyr Lys
            20                  25                  30
Gln Arg Val Lys Asn Lys
        35

<210> 6
<211> 52
<212> PRT
<213> Artificial Sequence

<220>
<223> Adrenomedullin

<400> 6
Tyr Arg Gln Ser Met Asn Asn Phe Gln Gly Leu Arg Ser Phe Gly Cys
1               5                   10                  15
Arg Phe Gly Thr Cys Thr Val Gln Lys Leu Ala His Gln Ile Tyr Gln
            20                  25                  30
Phe Thr Asp Lys Asp Lys Asp Asn Val Ala Pro Arg Ser Lys Ile Ser
        35                  40                  45
Pro Gln Gly Tyr
    50

<210> 7
<211> 13
<212> PRT
<213> Artificial Sequence

<220>
<221> MOD_RES
<222> 1
<223> ACETYLATION

<220>
<223> Alpha-Melanocyte Stimulating Hormone

<220>

<221> MOD_RES
<222> 13
<223> AMIDATION

<400> 7
Ser Tyr Ser Met Glu His Phe Arg Trp Gly Lys Pro Val
1               5                   10

<210> 8
<211> 23
<212> PRT
<213> Artificial Sequence

<220>
<223> Relaxin RLN1 subunit 1

<400> 8
Pro Tyr Val Ala Leu Phe Glu Lys Cys Cys Leu Ile Gly Cys Thr Lys
1               5                   10                  15
Arg Ser Leu Ala Lys Tyr Cys
            20

<210> 9
<211> 31
<212> PRT
<213> Artificial Sequence

<220>
<223> Relaxin RLN1 subunit 2

<400> 9
Val Ala Ala Lys Trp Lys Asp Asp Val Ile Lys Leu Cys Gly Arg Glu
1               5                   10                  15
Leu Val Arg Ala Gln Ile Ala Ile Cys Gly Met Ser Thr Trp Ser
            20                  25                  30

<210> 10
<211> 24
<212> PRT
<213> Artificial Sequence

<220>
<223> Relaxin RLN2 subunit 1

<400> 10
Gln Leu Tyr Ser Ala Leu Ala Asn Lys Cys Cys His Val Gly Cys Thr
1               5                   10                  15
Lys Arg Ser Leu Ala Arg Phe Cys
            20

<210> 11
<211> 29
<212> PRT
<213> Artificial Sequence

<220>
<223> Relaxin RLN2 subunit 2

<400> 11

Asp Ser Trp Met Glu Glu Val Ile Lys Leu Cys Gly Arg Glu Leu Val
1               5                   10                  15
Arg Ala Gln Ile Ala Ile Cys Gly Met Ser Thr Trp Ser
            20                  25

<210> 12
<211> 24
<212> PRT
<213> Artificial Sequence

<220>
<223> Relaxin RLN3 subunit 1

<400> 12
Asp Val Leu Ala Gly Leu Ser Ser Ser Cys Cys Lys Trp Gly Cys Ser
1               5                   10                  15
Lys Ser Glu Ile Ser Ser Leu Cys
            20

<210> 13
<211> 27
<212> PRT
<213> Artificial Sequence

<220>
<223> Relaxin RLN3 subunit 2

<400> 13
Arg Ala Ala Pro Tyr Gly Val Arg Leu Cys Gly Arg Glu Phe Ile Arg
1               5                   10                  15
Ala Val Ile Phe Thr Cys Gly Gly Ser Arg Trp
            20                  25

<210> 14
<211> 138
<212> PRT
<213> Artificial Sequence

<220>
<223> Interferon gamma

<400> 14
Gln Asp Pro Tyr Val Lys Glu Ala Glu Asn Leu Lys Lys Tyr Phe Asn
1               5                   10                  15
Ala Gly His Ser Asp Val Ala Asp Asn Gly Thr Leu Phe Leu Gly Ile
            20                  25                  30
Leu Lys Asn Trp Lys Glu Glu Ser Asp Arg Lys Ile Met Gln Ser Gln
        35                  40                  45
Ile Val Ser Phe Tyr Phe Lys Leu Phe Lys Asn Phe Lys Asp Asp Gln
    50                  55                  60
Ser Ile Gln Lys Ser Val Glu Thr Ile Lys Glu Asp Met Asn Val Lys
65                  70                  75                  80
Phe Phe Asn Ser Asn Lys Lys Lys Arg Asp Asp Phe Glu Lys Leu Thr
                85                  90                  95
Asn Tyr Ser Val Thr Asp Leu Asn Val Gln Arg Lys Ala Ile His Glu
            100                 105                 110
Leu Ile Gln Val Met Ala Glu Leu Ser Pro Ala Ala Lys Thr Gly Lys
        115                 120                 125
Arg Lys Arg Ser Gln Met Leu Phe Arg Gly
    130                 135

## Description

The present invention relates to human anti-inflammatory peptides for the inhalatory treatment of inflammatory pulmonary diseases. Advantageous features of an aerosol containing such human anti-inflammatory peptides and a method for producing said aerosol are disclosed. The present invention further relates to a kit for inhalatory treatment of inflammatory pulmonary diseases.

### BACKGROUND OF THE INVENTION

Pulmonary diseases affect the lower airways of the respiratory system, in particular the lungs. This term encompasses pathological conditions that impair the gas exchange in the lungs or bronchi in mammals. In general, they are differentiated into obstructive and restrictive pulmonary diseases. Obstructive pulmonary diseases are characterized by airway obstruction. This limits the amount of air that is able to enter the alveoli because of constriction of the bronchial tree, due to inflammation. Restrictive pulmonary diseases are characterized by a loss of lung compliance, causing incomplete lung expansion and increased lung stiffness.

They can be also categorized as airway diseases, lung tissue diseases, lung circulation diseases, lung infectious diseases and lung proliferative diseases. Airway diseases affect the tubes that carry oxygen and other gases into and out of the lungs. They usually cause a narrowing or blockage of the airways. Typical airway diseases include asthma, chronic obstructive pulmonary disease (COPD) and bronchiectasis. Lung tissue diseases affect the structure of the lung tissue. Scarring or inflammation of the tissue makes the lungs unable to expand fully. This complicates the gas exchange. As a result, these patients can't breathe deeply. Pulmonary fibrosis and sarcoidosis are typical examples thereof. Lung circulation diseases affect the blood vessels of the lung. They are caused by clotting, scarring, or inflammation of the blood vessels. They affect the gas exchange and possibly also heart function. A typical example is pulmonary hypertension. Lung infectious diseases refer to disorders caused by an infection of the lower airways, e.g. pneumonia. Lung proliferative diseases include all tumors or neoplasms of the lower airways.

Most of the airway diseases are caused by an underlying inflammation or at least include an inflammatory component. Lung tissue diseases have often also an inflammatory component, unless they are caused by direct physical impairment of the respiratory tract. Lung circulation diseases such as pulmonary hypertension have in general an inflammatory component at the affected vessel section. Infectious and proliferative diseases of the lungs may also have an inflammatory component, often secondary to the infection or the underlying malignancy.

Thus, these inflammatory pulmonary diseases have in common that they could be pharmacologically treated by anti-inflammatory drugs. However, therapeutic efficacy is often hampered by insufficient availability, respectively efficacy of the drug at the site of inflammation in the lung. Systemic administration, e.g. orally or parenterally, yields often no or only an insufficient therapeutic success.

An alternative administration route is inhalation. Metered-dose inhalers (MDI) are widely in use, e.g. in the treatment of asthma. They usually have a container, respectively canister for the pharmaceutical formulation, a metering valve, for metering the dispensed quantity and a mouthpiece for inhaling. The pharmaceutical formulation consists of the drug, a liquefied gas propellant such as hydrofluoroalkanes and optionally pharmaceutically acceptable excipients.

A specific group of MDIs are dry powder inhalers (DPI). They deliver the drug to the lungs in the form of a dry powder. Most DPIs rely on the force of patient inhalation to entrain powder from the device and subsequently break-up the powder into particles that are small enough to reach the lungs. For this reason, insufficient patient inhalation flow rates may lead to reduced dose delivery and incomplete disaggregation of the powder, leading to unsatisfactory device performance. Thus, most DPIs need a minimum inspiratory effort for proper use. Therefore, their use is limited to older children and adults.

While disorders affecting the bronchi or upper parts of the lower airways can be addressed this way, e.g. by asthma sprays, disorders affecting the alveoli where the gas exchange takes place can be only insufficiently treated because of ineffective inhalatory administration, e.g. COPD. The administered drug particles are not able to reach the bottom of the lungs by way of inhalation, at least not in a therapeutically effective amount.

Nebulizers use to administer the active principle in the form of a mist inhaled into the lungs. Physically, this mist is an aerosol. It is generated in the nebulizer by breaking up solutions and suspensions into small aerosol droplets that can be directly inhaled from the mouthpiece of the device. In conventional nebulizers the aerosol can be generated by mechanical force, e.g. spring force in soft mist nebulizers, or electrical force. In jet nebulizers a compressor brings oxygen or compressed air to flow at high velocity through the aqueous solution with the active principle, this way generating an aerosol. A variant are pressurized metered-dose inhalers (pMDIs). Ultrasonic wave nebulizers use an electronic oscillator that at high frequency causes vibration of a piezoelectric element for generating ultrasonic waves in the liquid reservoir with the active principle.

The most promising technology are vibrating mesh nebulizers. They use a mesh, respectively a polymer membrane having a very large number of laser-drilled holes. This membrane is placed between the liquid reservoir and the aerosol chamber. A piezoelectric element placed on the membrane induces high frequency vibrations of the membrane, leading to droplet formation in the aqueous solution and pressuring these droplets through the holes of the membrane into the aerosol chamber. With this technique very small droplet sizes can be generated. Moreover, a significantly shorter inhalation time for the patient can thus be achieved, a feature which drastically increases patient compliance. Only these mesh nebulizers are regarded to be able to generate liquid droplets with the active principle in the desired size range and bring them in a therapeutically effective amount into the patient's alveoli in a reasonable time.

However, not all agents that might be effective in the pharmaceutic treatment of inflammatory pulmonary diseases are suitable for the mesh nebulization technology. For example, some agents are nearly insoluble in water, or their intrinsic physicochemical molecule properties don't allow for the generation of an aerosol in the desired particle size range. Therefore, many nebulized anti-inflammatory agents met only mixed success in the treatment of inflammatory pulmonary diseases.

Thus, there is a medical need to find a pharmaceutical agent that shows a high efficacy in the treatment of inflammatory pulmonary diseases and at the same time allows for the generation of an aerosol in the desired particle size range in order to be able to reach the alveoli of a patient in need thereof.

Surprisingly, this task could be solved by the nebulization of selected human anti-inflammatory peptides.

### DETAILED DESCRIPTION OF THE INVENTION

The following human peptides were found to be outstandingly suitable for inhalatory application to a human suffering from a pulmonary disease:

### Vasoactive Intestinal Peptide (VIP)

VIP is a widely distributed human 28 amino acid neuropeptide. It belongs to a glucagon/secretin superfamily, being a ligand of class II G protein-coupled receptors (cf. Umetsu et al. (2011) Biochimica et Biophysica Acta 1814: 724-730). It exists in the two isoforms 1 and 2, having the same amino acid sequence. VIP is post-translationally cleaved from 170 amino acid VIP peptides isoform 1 preproprotein (125-152) and 169 amino acid VIP peptides isoform 2 preproprotein (124-151). In the scope of the present application the term VIP shall refer to both isoforms.

The amino acid sequence of human VIP is (from the N- to the C-terminus), as of GenelD 7432 and NP_003372.1 (isoform 1) and GenelD 7432 and NP_919416.1 (isoform 2), as of January 3rd, 2020:
His-Ser-Asp-Ala-Val-Phe-Thr-Asp-Asn-Tyr-Thr-Arg-Leu-Arg-Lys-Gln-Met-Ala-Val-Lys-Lys-Tyr-Leu-Asn-Ser-Ile-Leu-Asn (SEQ ID NO: 1).

VIP mediates a variety of physiological responses including gastrointestinal secretion (gastric acid, pancreatic juice, bile), relaxation of gastrointestinal vascular and respiratory smooth muscle activities, increase in bowel motility, coronary vasodilation accompanied by a positive inotropic and chronotropic effect, increase in vaginal lubrication, immune cell regulation, and apoptosis (cf. Bowen (1999) Vasoactive Intestinal Peptide. In: Pathophysiology of the Endocrine System: Gastrointestinal Hormones, Colorado State University; Bergman et al. (2009) Vasoactive Intestinal Peptide, In: Atlas of Microscopic Anatomy). Moreover, positive effects have been reported for VIP in respect of impotence, ischemia, dry eye, chronic inflammatory response syndrome (CIRS) and mental disorders such as Alzheimer's disease. VIP acts also as a synchronizing agent in the suprachiasmatic nucleus and thus interferes with circadian rhythms (Achilly (2016) J Neurophysiol 115: 2701-2704).

Under physiologic conditions VIP acts as a neuroendocrine mediator. The biological effects are mediated via specific receptors (VIP-R: VPAC1 and VPAC2) located on the surface membrane of various cells. Both receptors are G-protein-coupled receptors activating adenylate cyclase.

In the lung, VIP receptors have been detected on airway epithelium of the trachea and the bronchioles, in macrophages surrounding capillaries, in connective tissue of trachea and bronchi, in alveolar walls, and in the subintima of pulmonary veins and pulmonary arteries. Peptidergic nerve fibers are considered the source of VIP in the lung. VIP decreases the resistance in the pulmonary vascular system. It leads to sustained bronchodilation activity without remarkable cardiovascular side effects and is effective against disorders or diseases relating to bronchial spasms including asthma and any type of pulmonary hypertension.

VIP has potent anti-inflammatory properties. It inhibits the production of inflammatory cytokines and chemokines from macrophages, microglia and dendritic cells. VIP also reduces the expression of co-stimulatory molecules on antigen-presenting cells, and therefore reduces stimulation of antigen-specific CD4 T-cells. In terms of adaptive immunity, VIP promotes T-helper (Th)2-type responses and reduces inflammatory Th1-type responses (Gonzalez Rey and Delgado (2005) Curr Opin Investig Drugs 6: 1116-1123).

The VIP analogue Aviptadil is used in the treatment of erectile dysfunction.

As shown in Ex. 1, favorable FPM and MMAD values were measured for an aerosol generated from an aqueous solution containing VIP. This makes VIP a promising candidate for the inhalatory treatment of inflammatory pulmonary diseases.

### C-type Natriuretic Peptide (CNP)

CNP is a 22 amino acid human peptide that belongs to the family of the natriuretic peptides. In humans it is encoded by the NPPC (natriuretic peptide precursor C) gene that is responsible for the expression of the NPPC precursor protein. Post-translationally this peptide is cleaved to CNP.

The amino acid sequence of human CNP is (from the N- to the C-terminus), as of GenelD 4880 and NP_077720.1 105-126, as of January 2nd 2020:
Gly-Leu-Ser-Lys-Gly-Cys-Phe-Gly-Leu-Lys-Leu-Asp-Arg-Ile-Gly-Ser-Met-Ser-Gly-Leu-Gly-Cys (SEQ ID NO: 2).

Natriuretic peptides possess potent natriuretic, diuretic, and vasodilating activities and are implicated in body fluid homeostasis and blood pressure control. CNP does not have direct natriuretic activity. CNP is a selective agonist for the B-type natriuretic receptor (NPRB; synonym: NPR2; cf. Barr et al. (1996) Peptides 17: 1243-1251). It is synthesized and secreted from the vascular endothelium in response to growth factors, vascular injury, shear stress, nitric oxide and certain proinflammatory cytokines (cf. Suga et al. (1993) Endocrinology 133: 3038-3041; Chun et al. (1997) Hypertension 29: 1296-1302; Brown et al. (1997) Am J Physiol 272: H2919-H2931).

CNP is the most highly conserved of the natriuretic peptides between species. The major sites of expression are the nervous system, endothelial cells and the urogenital tract. CNP accounts for the biological activity of endothelium-derived hyperpolarizing factor. Its secretion is mediated through shear stress exerted on the endothelium wall. Several cytokines like tumor necrosis factor-a (TNF-alpha), interleukin-1 (IL-1) and transforming growth factor-beta (TGF-β) stimulate CNP expression. Besides its fundamental role in the regulation of vascular tone and local blood flow, CNP prevents smooth muscle proliferation, leukocyte recruitment and platelet aggregation. As such, CNP exerts a potent anti-atherogenic influence on blood vessel walls. CNP was found to improve the outcome in mice subjected to ischemia / reperfusion injury or myocardial infarction (Wang et al. (2007) Eur J Heart Fail 9: 548-557). Exogenous CNP attenuates lipopolysaccharide (LPS)-induced acute lung injury in mice (Kimura et al. (2015) J Surg Res 194: 631-637). CNP was found to ameliorate pulmonary fibrosis in mice (Kimura et al. (2016) Resp Res 17: 19).

CNP functions by interaction with membrane-bound guanylyl cyclase (GC-B), thus modulating cellular functions via the intracellular second messenger, cyclic GMP. Subsequent elevation of intracellular cGMP modulates the activity of specific downstream regulatory proteins such as cGMP-regulated phosphodiesterases (inhibition of PDE3 activity increases cAMP levels whereas stimulation of PDE2 enhances cAMP hydrolysis), ion channels and cGMP-dependent protein kinases type I (PKG I) and type II (PKG II). These third messengers, which are differentially expressed in different cell types, ultimately modify cellular functions.

As shown in Ex. 2, a favorable particle size distribution (FPM) and mass median aerodynamic diameter (MMAD) of an aerosol generated from an aqueous solution containing CNP was measured. This makes CNP a promising candidate for the inhalatory treatment of inflammatory pulmonary diseases.

### B-type Natriuretic Peptide (BNP, Brain Natriuretic Peptide)

BNP is a 32 amino acid human peptide that also belongs to the family of the natriuretic peptides. It is attached to a 76 amino acid fragment 1 - 134 attached to the N-terminus in the prohormone called NT-proBNP (BNPT). Post-translationally BNPT is cleaved to BNP.

The amino acid sequence of human BNP is (from the N- to the C-terminus), as of GenelD 4879 and NP_002512.1 103-134, as of January 3rd, 2020:
Ser-Pro-Lys-Met-Val-Gln-Gly-Ser-Gly-Cys-Phe-Gly-Arg-Lys-Met-Asp-Arg-Ile-Ser-Ser-Ser-Ser-Gly-Leu-Gly-Cys-Lys-Val-Leu-Arg-Arg-His (SEQ ID NO: 3).

NPRA (synonym: NPR1; Natriuretic Peptide Receptor-A) is the principal receptor of BNP, to a much lesser extent it binds to NPRB (cf. Miyagi et al. (2000) Eur J Biochem 267: 5758-5768). The physiologic actions of BNP include a decrease in systemic vascular resistance and central venous pressure as well as an increase in natriuresis. This results in a decrease in blood pressure due to the decrease in systemic vascular resistance. BNP diminishes the cardiac output due to an overall decrease in central venous pressure and preload as a result of the reduction in blood volume that follows natriuresis and diuresis. BNP activation of NPRA leads to the inhibition of cardiac fibrosis in mice (Tamura et al. (2000) PNAS 97: 4238-4244). BNP was found to be a prognostic marker for pulmonary hypertension in chronic lung disease such as COPD and DPLD (diffuse parenchymal lung diseases; cf. Leuchte et al. (2006) Am J Respir Crit Care Med 173: 744-750). Serum concentrations of NT-proBNP (respectively BNP) are a useful parameter for assessing pulmonary hypertension in patients with end-stage lung disease referred to lung transplantation (Nowak et al. (2018) Transplant Proc 50: 2044-2047).

BNP is produced in the heart and secreted by the cardiac atria and above all the cardiac ventricles. NPRA is expressed in kidney, lung, adipose, adrenal, brain, heart, testis and vascular smooth muscle tissue (cf. Goy et al. (2001) Biochem J 358: 379-387).

BNP exerts its biological action via binding to a specific receptor, a specific guanyl cyclase (GC-A), activating an increase of cyclic guanosine monophosphate (cGMP) levels that mediates the vasodilatory properties via activation of distinct protein kinases (cf. Yasue et al. (1994) Circulation 90: 195-203). Increased pulmonary artery pressures leads to a highly induced BNP expression under hypoxic conditions *in vivo* in order to reduce pulmonary vascular resistance. BNP inhibits the secretion of IL-1β via downregulation of NF-κB and Caspase-1 activation in human monocytes (Mezzasoma et al. (2017) Mediators Inflamm: 5858315). Thus, BNP shows also anti-inflammatory actions.

Recombinant BNP (Nesiritide) failed to show a beneficial effect in a clinical trial for acute decompensated heart failure (O'Connor et al. (2011) New Engl J Med 365: 32-43).

As shown in Ex. 3, favorable FPM and MMAD values were measured for an aerosol generated from an aqueous solution containing BNP. This makes BNP a promising candidate for the inhalatory treatment of inflammatory pulmonary diseases.

### Pituitary Adenylate Cyclase-activating Peptide (PACAP)

PACAP is a human neuropeptide that exists in two variants, PACAP-27 and PACAP-38. They are differentially excised from the precursor peptide adenylate cyclase activating polypeptide 1 (ADCYAP1; cf. Hosoya et al. (1992) Biochim Biophys Acta 1129: 199-206). Both variants display the same physiological actions. In the scope of the present application the term PACAP shall refer to PACAP-27 as well as to PACAP-38.

The amino acid sequences of human PACAP are (from the N- to the C-terminus), as of GenelD 116 and NM_001099733 132-158 (variant 1, PACAP-27) and GenelD 116 and NP_001108.2 132-169 (variant 2, PACAP-38), as of January 3rd, 2020:
PACAP-27: His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu (SEQ ID NO: 4), and
PACAP-38: His-Ser-Asp-Gly-Ile-Phe-Thr-Asp-Ser-Tyr-Ser-Arg-Tyr-Arg-Lys-Gln-Met-Ala-Val-Lys-Lys-Tyr-Leu-Ala-Ala-Val-Leu-Gly-Lys-Arg-Tyr-Lys-Gln-Arg-Val-Lys-Asn-Lys (SEQ ID NO: 5).

PACAP is a very potent stimulator of adenylate cyclase and therefore increasing adenosine 3,5-cyclic monophosphate (cAMP) in various cells (cf. Miyata et al. (1989) Biochem Biophys Res Comm 161: 567-574). It functions as a hypothalamic hormone, neurotransmitter, neuromodulator, vasodilator and neurotrophic factor. PACAP plays an important role in the endocrine system as a potent secretagogue for adrenaline from the adrenal medulla. Further, PACAP is a neurotrophic factor during brain development. In the adult brain, PACAP functions as a neuroprotective factor that attenuates neuronal damage resulting from various insults. PACAP is widely distributed in the brain and peripheral organs, notably in the endocrine pancreas, gonads, and respiratory tracts.

Molecular cloning of PACAP receptors has shown the existence of three distinct receptor subtypes. These are the PACAP-specific PAC1 receptor (Pisegna and Wank (1993) PNAS 90: 6345-6349), which is coupled to several transduction systems, and the two VPAC1 (Ishihara et al. (1991) EMBO J 10: 1635-1641) and VPAC2 (Lutz et al. (1993) FEBS Lett 334: 3-8) receptors, which are primarily coupled to adenylyl cyclase. PAC1 receptors are particularly abundant in the brain and pituitary and adrenal glands whereas VPAC receptors are expressed mainly in the lungs (cf. Busto et al. (2000) Peptides 21: 265-269). PACAP regulates the vascular tone in vessels, which is orchestrated by a complex network of vasoactive effector substances produced either locally in the endothelium, in vascular smooth muscle cells (VSMC), in extrinsic and intrinsic nerves, and by the vascular blood flow itself.

PACAP-27 showed an inhibition of bronchoconstriction in guinea pigs *in vivo* (Linden et al. (1995) Br J Pharmacol 115: 913-916). PACAP-38 is present in lung and constitutes a potent endogenous bronchodilator through inhibition of smooth muscle contraction induced by cholinergic and excitatory non-adrenergic and non-cholinergic nerves (Yoshida et al. (2000) Eur J Pharmacol 39: 77-83). PAC1 receptor-deficient mice were found to develop pulmonary hypertension and right heart failure (Otto et al. (2004) Circulation 110: 3245-3251).

Inhibition of the pro-inflammatory cytokines TNF-alpha and IL-6 by PACAP protected mice from lethal endotoxemia (Delgado et al. (1999) J Immunol 162: 1200-1205). PACAP plays an anti-inflammatory role in endotoxin-induced airway inflammation in mice in vivo (Elekes et al. (2011) Peptides 32: 1439-1446).

Intravenously infused PACAP-38 was recently found to trigger delayed migraine-like headaches in most subjects who experience migraine headaches (Wachek et al. (2018) J Headache Pain 19: 23).

As shown in Ex. 4, favorable FPM and MMAD values were measured for an aerosol generated from an aqueous solution containing PACAP-38. This makes PACAP a promising candidate for the inhalatory treatment of inflammatory pulmonary diseases.

### Adrenomedullin (ADM, AM)

Adrenomedullin consists of 52 amino acids. It is a member of the Calcitonin Gene-Related Peptide (CGRP) family, which was originally discovered in 1993 in human adrenal medulla as a hypotensive factor produced by pheochromocytoma cells. It is enzymatically cleaved from the 185 amino acid precursor protein preproadrenomedullin.

The amino acid sequence of human adrenomedullin is (from the N- to the C-terminus), as of GenelD 133 and NP_001115.1 95-146, as of January 3rd, 2020:
Tyr-Arg-Gln-Ser-Met-Asn-Asn-Phe-Gln-Gly-Leu-Arg-Ser-Phe-Gly-Cys-Arg-Phe-Gly-Thr-Cys-Thr-Val-Gln-Lys-Leu-Ala-His-Gln-Ile-Tyr-Gln-Phe-Thr-Asp-Lys-Asp-Lys-Asp-Asn-Val-Ala-Pro-Arg-Ser-Lys-Ile-Ser-Pro-Gln-Gly-Tyr (SEQ ID NO: 6).

Adrenomedullin is a widely expressed, including the vasculature, lungs, heart and adipose tissue. Both constitutive and induced secretion has been demonstrated from endothelial cells, vascular smooth muscle cells, cardiac myocytes, leucocytes, fibroblasts, or adipocytes (cf. Ichiki et al. (1994) FEBS Lett 338: 6-10).

The actions of Adrenomedullin are mediated by the 7-transmembrane G-protein-coupled calcitonin receptor-like receptor (CRLR), which co-assembles with subtypes 2 and 3 of a family of receptor-activity-modifying proteins (RAMPs; synonym: AM1 and AM2, respectively; cf. Kamitani et al. (1999) FEBS Lett 448: 111-114). The receptor component factor (RCF) has been shown to be essential for signal transduction of Adrenomedullin, and to interact with CRLR directly within the cells. A functional Adrenomedullin receptor, which consists of at least three proteins: CRLR, RAMP, and RCF, couples the receptor to the intracellular signal transduction pathway. These receptors are abundantly expressed on alveolar capillaries and in pulmonary microvascular endothelial cells. Lungs contain specific Adrenomedullin-binding sites at a density much higher than in any other organ studied.

Adrenomedullin stimulates its receptors to increase the production of cAMP and nitric oxide (cf. Hay et al. (2006) Pharmacol Ther 109: 173-197). cAMP/protein kinase in smooth muscle cells regulates the vasodilatory effects of Adrenomedullin. In endothelial cells, vasodilation predominantly occurs by an eNOS/NO pathway. Adrenomedullin induces Akt activation in the endothelium via the Ca²⁺/calmodulin-dependent pathway. This is implicated in the production of nitric oxide, which in turn induces endothelium-dependent vasodilation. Adrenomedullin has a potent protective, anti-apoptotic role through the PI3K/Akt pathway. GSK-3β is a downstream protein kinase of Akt, which when phosphorylated, causes inactivation and reduced caspase signaling. The Adrenomedullin-mediated anti-apoptotic effect is associated with increased GSK-3β signaling. The angiogenic effect of Adrenomedullin is mediated by activation of Akt as well as MAPK/ERK 1/2 and FAK in endothelial cells. The MAPK-ERK signaling has also well-characterized stress-induced protective effects. Adrenomedullin triggers rapid ERK activation, which has anti-apoptotic and compensatory hypertrophic effects and stimulates smooth muscle cell proliferation.

As a consequence of the widespread expression of the peptide and its receptors, the peptide participates in the control of central body functions, such as vascular tone regulation, fluid and electrolyte homeostasis or regulation of the reproductive system.

Adrenomedullin stimulating angiogenesis and increases the tolerance of cells to oxidative stress and hypoxic injury. Adrenomedullin is seen as a positive influence in diseases such as hypertension, myocardial infarction, COPD and other cardiovascular diseases.

Pro-inflammatory cytokines, such TNF-alpha and IL-1, and lipopolysaccharides, induce the production and secretion of Adrenomedullin. In consequence it induces the downregulation these inflammatory cytokines are downregulated in cultured cells (cf. Isumi et al. (1999) FEBS Lett 463: 110-114). Adrenomedullin is seen as a potential therapeutic agent for inflammatory bowel disease (cf. Ashizuka et al. (2013) Curr Protein Pept Sci 14: 246-255).

Adrenomedullin was found to ameliorate lipopolysaccharide-induced acute lung injury in rats (cf. Itoh et al. (2007) Am J Physiol Lung Cell Mol Physiol 293: L446-452). Adrenomedullin also attenuates ventilator-induced lung injury in mice (Müller et al. (2010) Thorax 65: 1077-1084). Adrenomedullin and adrenomedullin binding protein-1 prevented acute lung injury after gut ischemia-reperfusion (Dwivedi et al. (2007) J Am Coll Surg 205: 284-289). Anti-inflammatory effects of adrenomedullin on acute lung injury induced by carrageenan were observed in mice (Talero et al. (2012) Mediators Inflamm: 717851).

As shown in Ex. 5, favorable FPM and MMAD values were measured for an aerosol generated from an aqueous solution containing adrenomedullin. This makes adrenomedullin a promising candidate for the inhalatory treatment of inflammatory pulmonary diseases.

### α-Melanocyte Stimulating Hormone (a-MSH)

α-MSH is a peptide hormone and neuropeptide of the melanocortin family. Human α-MSH consists of 13 amino acids.

α-MSH is generated as a proteolytic cleavage product from adrenocorticotropic hormone (ACTH (1-13)), which is in turn a cleavage product of proopiomelanocortin (POMC (138-150)).

The amino acid sequence of human α-MSH is (from the N- to the C-terminus), as of GenelD 5443 and UniProtKB - P01189, as of January 7th, 2020:
Ac-Ser-Tyr-Ser-Met-Glu-His-Phe-Arg-Trp-Gly-Lys-Pro-Val-NH₂ (SEQ ID NO: 7).

α-MSH is produced in the anterior lobe of the pituitary gland, neurons, T lymphocytes, macrophages, skin cells, endothelial cells, and in placenta cells. α-MSH exerts its functions by activating specific melanocortin receptors (MC1, MC3, MC4, MC5), all of which belong to the G protein-coupled receptor (GPCR) family. Upon α-MSH binding, the α-subunit of the receptor-coupled stimulatory G protein (Gsa) activates adenylate cyclase, which increases cAMP production in the target cell, which in turn activates protein kinase A (PKA), causing four main effects:
PKA activation induces the phosphorylation of the cAMP-responsive-element-binding protein (CREB), which, owing to its high affinity for the co-activator CREB-binding protein (CBP), prevents the association of CBP with p65 (which is a key component of nuclear factor-kappa B (NF-κB)).

Activated PKA inhibits IkappaB kinase (IκK), which stabilizes the IκB inhibitor and prevents nuclear translocation of p65.

PKA activation inhibits MAPK/ERK kinase kinase 1 (MEKK1) phosphorylation and activation, and the subsequent activation of p38 and TATA-binding protein (TBP). Non-phosphorylated TBP lacks the ability to bind to the TATA box and to form an active transactivating complex with CBP and NF-κB. A reduction in the amounts of nuclear p65, CBP and phosphorylated TBP inhibits the formation of the conformationally active transactivating complex that is required for the transcription of most pro-inflammatory cytokine and chemokine genes.

Inhibition of MEKK1 by PKA subsequently deactivates JUN kinase (JNK) and cJUN phosphorylation. The composition of the activator protein 1 (AP1) complex changes from the transcriptionally active cJUN-cJUN, to the transcriptionally inactive JUNB-cFOS or CREB.

Thus, the transcription of several pro-inflammatory mediators (e.g. TNF-alpha, IL-1, IL-6 and IL-8, Groα, KC), is significantly disrupted by treatment with α-MSH (cf. Manna et al. (1998) J Immunol 161: 2873-2380). In α-MSH vector-transfected human pulmonary epithelial cells NF-κB is also inhibited (Ichiyama et al. (2000) Peptides 21: 1473-1477). Non-cytokine pro-inflammatory parameters such as nitric oxide, PGE2 and reactive oxygen species as well as adhesion molecules such as ICAM-1, CD40, CD86, VCAM-1 and E-selectin are likewise suppressed (cf. Wang et al. (2019) Frontiers in Endocrinology 10: 683).

Via MCR1 α-MSH stimulates the production and release of melanin by melanocytes in skin and hair. In the hypothalamus α-MSH suppresses appetite and contributes to sexual arousal (cf. King et al. (2007) Curr Top Med Chem 7: 1098-1106). It has a role in cellular energy homeostasis. Further, it shows protective features against ischemia and reperfusion injury (Varga et al. (2013) J Molec Neurosci 50: 558-570). Specific dilatative effects of α-MSH on the coronary vasculature and aortic rings were reported as an indicator for protection against cardiovascular ischemia / reperfusion (I/R) injury (Vecsernyes et al. (2017) J Cardiovasc Pharmacol 69: 286-297).

Anti-inflammatory treatment with α-MSH showed promising results in experimental rheumatoid arthritis in rats (Ceriani et al. (1994) Neuroimmunomodulation 1: 28-32), systemic inflammation such as sepsis, septic shock and acute respiratory distress syndrome (ARDS). In a bleomycin-induced acute lung injury model in rats α-MSH showed to be beneficial (Colombo et al. (2007) Shock 27: 326-333).

As shown in Ex. 6, favorable FPM and MMAD values were measured for an aerosol generated from an aqueous solution containing α-MSH. This makes α-MSH a promising candidate for the inhalatory treatment of inflammatory pulmonary diseases.

### Relaxin

Relaxin is a human peptide hormone that belongs to the relaxin-like peptide family and encompasses isoforms relaxin-1 (RLN1), relaxin-2 (RLN2) and relaxin-3 (RLN3). For each relaxin a heterodimer is differentially cleaved from 185 amino acid precursor hormones (RLN1: prorelaxin H1 and RLN2: prorelaxin H2) or a 142 amino acid precursor hormone (RLN3: prorelaxin H3).

RLN1 encompasses 54 amino acids (subunit 1: 23 (163-185); subunit 2: 31 (23-53)). RLN2 encompasses 53 amino acids (subunit 1: 24 (162-185); subunit 2: 29 (25-53)). RLN3 encompasses 51 amino acids (subunit 1: 24 (119-142); subunit 2: 27 (26-52)).

All relaxin variants display the same physiological actions. In the scope of the present application the term relaxin shall refer to RLN1, RLN2 as well as RLN3.

The amino acid sequences of human relaxin are (from the N- to the C-terminus), as of GenelD 6013 and UniProt 04808 (RLN1), GenelD 6019 and UniProt 04090 (RLN2) and GenelD 117579 and UniProt Q8WXF3 (RLN3), as of January 8th, 2020:
RLN1 subunit 1: Pro-Tyr-Val-Ala-Leu-Phe-Glu-Lys-Cys-Cys-Leu-Ile-Gly-Cys-Thr-Lys-Arg-Ser-Leu-Ala-Lys-Tyr-Cys (SEQ ID NO: 8)
RLN1 subunit 2: Val-Ala-Ala-Lys-Trp-Lys-Asp-Asp-Val-Ile-Lys-Leu-Cys-Gly-Arg-Glu-Leu-Val-Arg-Ala-Gln-Ile-Ala-Ile-Cys-Gly-Met-Ser-Thr-Trp-Ser (SEQ ID NO: 9)
RLN2 subunit 1: Gln-Leu-Tyr-Ser-Ala-Leu-Ala-Asn-Lys-Cys-Cys-His-Val-Gly-Cys-Thr-Lys-Arg-Ser-Leu-Ala-Arg-Phe-Cys (SEQ ID NO: 10)
RLN2 subunit 2: Asp-Ser-Trp-Met-Glu-Glu-Val-Ile-Lys-Leu-Cys-Gly-Arg-Glu-Leu-Val-Arg-Ala-Gln-Ile-Ala-Ile-Cys-Gly-Met-Ser-Thr-Trp-Ser (SEQ ID NO: 11)
RLN3 subunit 1: Asp-Val-Leu-Ala-Gly-Leu-Ser-Ser-Ser-Cys-Cys-Lys-Trp-Gly-Cys-Ser-Lys-Ser-Glu-Ile-Ser-Ser-Leu-Cys (SEQ ID NO: 12)
RLN3 subunit 2: Arg-Ala-Ala-Pro-Tyr-Gly-Val-Arg-Leu-Cys-Gly-Arg-Glu-Phe-Ile-Arg-Ala-Val-Ile-Phe-Thr-Cys-Gly-Gly-Ser-Arg-Trp (SEQ ID NO: 13).

Relaxin is produced primarily by the corpus luteum in both pregnant and nonpregnant females. It attains the highest plasma levels during pregnancy. In males, relaxin is synthesized in the prostate and released in the seminal fluid. An additional source of relaxin is the heart atrium.

Relaxin acts on a group of four G protein-coupled receptors known as Relaxin Family Peptide (RXFP) Receptors. The leucine-rich repeat containing RXFP1 and RXFP2 and the small peptide-like RXFP3 and RXFP4 are the physiological targets for relaxin. Activation of RXFP1 or RXFP2 causes increased production of second messenger cAMP (cf. Hsu et al. (2002) Science 295: 671-674).

In the cardiovascular system, relaxin works mainly by activating the nitric oxide pathway. Other mechanisms include activation of NF-κB leading to vascular endothelial growth factor (VEGF) and matrix metalloproteinases transcription (Raleigh et al. (2016) J Cardiovasc Pharmacol Therap 21: 353-362).

Relaxin has several physiological functions such as induction of collagen remodeling, softening of the tissue of the birth cannel, inhibition of uterine contractile activity, or stimulation, growth and differentiation of mammary gland. In the lungs, relaxin can regulate excessive collagen deposition in disease states such as pulmonary fibrosis and pulmonary hypertension. Further it has a regulatory function for the cardiovascular system by dilating systemic resistance arteries (Raleigh et al. (2016) J Cardiovasc Pharmacol Therap 21: 353-362).

Relaxin activating RXFP1 has the potential for the treatment of diseases involving tissue fibrosis such as pulmonary fibrosis, cardiac failure, renal failure, asthma, fibromyalgia and scleroderma (cf. van der Westhuizen et al. (2007) Current Drug Targets 8: 91-104) and may also be useful to facilitate embryo implantation.

Relaxin protects against airway remodeling changes occurring in asthma and potentially also in COPD (Tang et al. (2009) Ann N Y Acad Sci 1160: 342-247).

Relaxin treatment of human lung fibroblasts resulted in a reduction in the expression of collagen types I and III and fibronectin in response to TGF-β, a potent fibrogenic agent, and furthermore promoted extracellular matrix degradation by increasing the levels of matrix metallopeptidases. In an *in vivo* model, relaxin treatment dramatically decreased bleomycin-induced collagen content in the lung, alveolar thickening, and improved the overall fibrosis score (Unemori et al. (1996) J Clin Invest 98: 2379-2745).

Relaxin was recently found to reverse inflammatory and immune signals in aged hearts (Martin et al. (2018) PloS One 13: e0190935). The anti-inflammatory properties of relaxin have been reviewed in Nistor et al. (2018) Neural Regen Res 13: 402-405).

As shown in Ex. 7, favorable FPM and MMAD values were measured for an aerosol generated from an aqueous solution containing relaxin. This makes relaxin a promising candidate for the inhalatory treatment of inflammatory pulmonary diseases.

### Interferon gamma (IFN-γ)

IFN-γ is a human cytokine with a molecular weight of 17kD that exerts a wide range of biological effects. It encompasses 138 amino acids and is cleaved from a 166 amino acid propeptide (24-161).

The amino acid sequence of human IFN-γ is (from the N- to the C-terminus), as of GenelD 3458 and UniProt P01579, as of January 8th, 2020:
Gln-Asp-Pro-Tyr-Val-Lys-Glu-Ala-Glu-Asn-Leu-Lys-Lys-Tyr-Phe-Asn-Ala-Gly-His-Ser-Asp-Val-Ala-Asp-Asn-Gly-Thr-Leu-Phe-Leu-Gly-Ile-Leu-Lys-Asn-Trp-Lys-Glu-Glu-Ser-Asp-Arg-Lys-Ile-Met-Gln-Ser-Gln-Ile-Val-Ser-Phe-Tyr-Phe-Lys-Leu-Phe-Lys-Asn-Phe-Lys-Asp-Asp-Gln-Ser-Ile-Gln-Lys-Ser-Val-Glu-Thr-Ile-Lys-Glu-Asp-Met-Asn-Val-Lys-Phe-Phe-Asn-Ser-Asn-Lys-Lys-Lys-Arg-Asp-Asp-Phe-Glu-Lys-Leu-Thr-Asn-Tyr-Ser-Val-Thr-Asp-Leu-Asn-Val-Gln-Arg-Lys-Ala-Ile-His-Glu-Leu-Ile-Gln-Val-Met-Ala-Glu-Leu-Ser-Pro-Ala-Ala-Lys-Thr-Gly-Lys-Arg-Lys-Arg-Ser-Gln-Met-Leu-Phe-Arg-Gly (SEQ ID NO: 14).

IFN-γ binds to a heterodimeric receptor consisting of Interferon gamma receptor 1 (IFNGR1) and Interferon gamma receptor 2 (IFNGR2) thus activating the JAK-STAT pathway. It further binds to glycosaminoglycan heparan sulfate (HS) at the cell surface (Sadir et al. (1998) J Biol Chem 273: 10919-10925).

IFN-γ is produced predominantly by natural killer (NK) and natural killer T (NKT) cells as part of the innate immune response, and by CD4 Th1 and CD8 cytotoxic T lymphocyte (CTL) effector T cells once antigen-specific immunity develops. The anti-inflammatory effects of IFN-γ are mainly mediated by immunostimulation and immunomodulation (cf. Schoenborn et al. (2007) Advances in Immunology 96: 41-101).

Those effects include the induction of MHC class II antigens improving antigen presentation, macrophage activation, increased immunoglobulin production from B lymphocytes, enhanced NK cell activity, the later effects aiming to facilitate killing of intracellular pathogens.

The anti-fibrotic properties of IFN-γ comprise inhibition of TGF-β-induced cellular signaling via activation of signal transducer and activator of transcription (STAT)-1. Studies of lung tissue and blood from patients with idiopathic pulmonary fibrosis (IPF) demonstrated absolute and relative deficits of IFN-γ compared to Th2 cytokines. Lack of IFN-γ expression in lung fibroblasts fosters the stimulated profibrotic effects of the TGF-β pathway. The overexpression of CD154 (a CD40 ligand) has been shown to be a hallmark of fibrotic lung fibroblasts. This CD154 overexpression has been shown to be markedly reduced by IFN-γ in fibrotic lung fibroblasts derived from IPF patients. In addition, the IFN-γ-inducible chemokines CXCL9, CXCL10 (IP-10), and CXCL11 use the receptor CXCR3 for their biological activity. Lack or downregulation of either CXCL10 or CXCL11 chemokines, or the CXCR3 chemokine receptor is clearly associated with progression of pulmonary fibrosis. Addition of IFN-γ induces the expression of the missing factors CXCL10, CXCL11, reverts the fibrotic phenotype caused by CXCR3 deficiency, and therefore IFN-γ reduces pulmonary fibrosis development. Finally, the classical activation of alveolar macrophages by IFN-γ leads to inhibition of fibrogenesis of fibroblasts by releasing antifibrogenic or fibrolytic factors.

Recombinant human IFN-γ has been expressed in different expression systems. Human IFN-γ is commonly expressed in *Escherichia coli,* marketed as ACTIMMUNE®. It is approved by the FDA for the treatment of chronic granulomatous disease and osteopetrosis (cf. Todd et al. (1992) Drugs 43: 111-122). A common off-label use is in the treatment of severe atopic dermatitis (cf. Akhavan et al. (2008) Seminars in Cutaneous Medicine and Surgery 27: 151-155).

As shown in Ex. 8, favorable FPM and MMAD values were measured for an aerosol generated from an aqueous solution containing IFN-γ. This makes IFN-γ a promising candidate for the inhalatory treatment of inflammatory pulmonary diseases.

All these human peptides according to the invention have been described in animal models to have beneficial effects on inflammatory pulmonary diseases. As of now, their therapeutic use was hampered by the difficulties to make these peptides biologically available at the target sites in the lung in a concentration that is effective in the treatment or the prophylaxis of the inflammatory pulmonary diseases according to the invention.

Therefore, the terms "peptides according to the invention", respectively "a peptide according to the invention" refer to vasoactive intestinal peptide, C-type natriuretic peptide, B-type natriuretic peptide, pituitary adenylate cyclase-activating peptide, adrenomedullin, alpha-melanocyte stimulating hormone, relaxin and/or interferon gamma.

From a pharmacokinetic point of view or for a production rationale it may be preferable to use a prodrug as a dosage form. A prodrug is administered in a pharmacologically inactive form and is metabolically converted into the active form inside the body. This conversion may occur systemically or locally. Thus, the present patent application refers also to prodrugs of the peptides according to the invention. In particular, it refers also to unprocessed, respectively not cleaved propeptides of the peptides according to the invention.

In the scope of this application an aerosol is a mixture of air and solid or liquid particles. In particular, the term "aerosol containing a peptide according to the invention" refers to an aerosol that has been generated by nebulization of an aqueous solution containing a peptide according to the invention.

Unless otherwise stated, any technical or scientific term used in the present invention has the meaning that a man skilled in the relevant technical art will attribute to them.

According to the application the terms "drug substance", "active substance", "active agent", "pharmaceutically active agent", "active ingredient" or "active pharmaceutical ingredient" (API) refer to one or more of the human anti-inflammatory peptides according to the invention, if not stated otherwise or used in a general sense.

The terms "composition" or "pharmaceutical composition" comprise at least one active ingredient in any pharmacologically acceptable defined dosage and dosage form together with at least one pharmaceutically acceptable excipient, as well as all agents that are generated from the ingredients as outlined below directly or indirectly as a combination, accumulation, complex or crystal, or as a consequence of other reactions or interactions, as well as optionally at least one further pharmaceutical drug, as listed below.

The term "excipient" is used in this application to describe any component of a pharmaceutical composition apart of the pharmaceutically active principle. The selection of suitable excipients depends on a variety of factors, such as the dosage form, the dosage, the desired solubility and the stability of the composition.

The terms "effect", "therapeutic effect", "action", "therapeutic action", "efficacy" and "effectiveness" in regard to the substance of the invention or any other active substance mentioned in the description refers to causally occurring beneficial consequences in the organism to which said substance has been administered before.

According to the invention the terms "effective amount" and "therapeutically effective amount" refer to an amount of the substance of the invention that is sufficiently large to cause a desired beneficial effect in a subject in need of such a treatment.

The terms "treatment" and "therapy" comprise the administration of at least the substance of the invention, alone or in combination with at least one further pharmaceutical drug, independently of the chronological order of the administration. Such an administration is intended to substantially improve the disease course of an inflammatory pulmonary disease by either completely curing the disease or by stopping or decelerating the increase of disabilities during the course of the disease.

The terms "prophylaxis" or "prophylactic treatment" comprise the administration of at least the substance of the invention, alone or in combination with at least one further pharmaceutical drug, independently of the chronological order of the administration, in order to prevent or suppress the manifestation of symptoms attributed to an inflammatory pulmonary disease. It refers in particular to medical conditions of a patient in which the manifestation of such symptoms is expected to occur in the far or near future with a reasonable probability.

The terms "subject" and "patient" comprise individuals suffering from disease symptoms or disabilities related to an inflammatory pulmonary disease wherein said diagnosis is either approved or suspected. Individuals are mammals, in particular humans.

In the scope of the present application the term "medicine" shall comprise human and veterinary medicine.

In the sense of the present patent application the terms "inflammatory diseases" or "inflammatory pulmonary diseases" refer to diseases, disorders or other body conditions in which an inflammation, in particular of the lungs, becomes manifest as a major symptom. An inflammation is the response of body tissues to irritation (exogenous or endogenous noxae) or injury. It can be provoked amongst others by physical, chemical and biologic stimuli, comprising mechanical trauma, radiation damage, corrosive chemicals, extremes of heat or cold, infectious agents such as bacteria, viruses, fungi and other pathogenic microorganisms or parts of them. An inflammation can have beneficial (e.g. within the scope of wound healing) and/or detrimental effects in the affected tissue(s). At a first stage an inflammation is regarded as acute. When it isn't terminated after some time the inflammation may become chronic. Typical signs of an inflammation are redness, swelling, heat development, pain and reduced functionality. This may even lead to a loss of function of the affected tissue.

An inflammation is one of the first responses of the immune system that has become activated e.g. by an infection or degenerated endogenous cells. The system of innate immunity mediates an unspecific response, amongst others a general inflammatory response, while the adaptive immune system provides reactions specific to the respective pathogen, which will then be remembered by the immune system. An organism can be in an immunodeficient state, i.e. the immune response is not able to cope with the aforementioned irritations or injuries in a satisfactory manner. On the other hand, the immune system might become hyperactive and turn its defense against endogenous tissues, as in the case of autoimmune diseases.

In the sense of the present patent application the terms "degenerative diseases" or "degenerative pulmonary diseases" refer to diseases, disorders or other body conditions in which a continuous process leads to degenerative cell changes. The affected tissues or organs deteriorate continuously over time. Such a degeneration may be due to physical or physiological over-exercise of specific vulnerable body structures, lifestyle, eating habits, age, congenital diseases or other endogenous causes. The degeneration can be caused or accompanied by an atrophy or dystrophy of the respective tissue or organ, especially the lungs. Often a loss of function and/or an irreversible damage of the affected tissue or organ occurs.

In the sense of the present patent application the terms "lesion", "microlesion" and "trauma" refer to injuries of different size and scope in the affected pulmonary tissue. They can be inflicted by spontaneous physical impact in which the impacting force or torque leads to a tissue damage. But they can also be the final consequence of a previous degenerative disease of the affected pulmonary tissue, or vice versa a microlesion may be the starting point of such a degenerative disease in the wake of the microlesion. Also an inflammation of the affected pulmonary tissue can favor such a microlesion or trauma, or it can be their sequelae. So these terms are interconnected with inflammation and degenerative disease.

In the sense of the present patent application the term "primary" disease, as e.g. a "primary inflammatory or degenerative disease" refers to pulmonary diseases which are not autoimmune-mediated.

If it is known that a healthy person is or will be vulnerable to inflammatory or degenerative diseases, or a tissue damage is to be expected due to a constant overstrain of the respective tissue or organ it can be indicated to give a prophylactic medication in order to prevent or at least to mitigate the expected impairment or damage. Thus, the present patent application refers also to a prophylactic use according to the invention.

There are also cases in which an inflammatory pulmonary disease results in a degenerative disease. Examples therefore will be given further below. Thus, the present patent application refers to the use according to the invention in the prophylaxis and/or treatment of inflammatory and/or degenerative pulmonary diseases, particularly in the treatment of primary inflammatory and/or degenerative pulmonary diseases.

In the scope of the present application the term "pulmonary" refers to organs and tissues of the lower respiratory tract. Examples of organs and tissues of the lower respiratory tract are, without being limiting, the lungs including their lobes, apices, lingulae and alveoli; the bronchi including respiratory bronchioles; tracheal and bronchi rings including the carina; pulmonary vessels including lung vessels and bronchial vessels and bronchial vessels; bronchopulmonary lymph nodes; autonomous nervous system of the lung;

In the scope of the present application the "pulmonary" further refers to adjacent organs and tissues that functionally or structurally are closely linked to the lower respiratory tract and/or the thorax and therefore can be pharmaceutically accessed excellently by inhalation. Examples are, without being limiting, pleura, diaphragm, pulmonary artery and pulmonary vein.

In the scope of the present application the terms "alveoli" and "alveolar" refer to the tissue structures at the bottom of the lung airways. Alveoli are hollow cup-shaped cavities found in the lung parenchyma where gas exchange takes place. Further, they are located sparsely on the respiratory bronchioles, line the walls of the alveolar ducts, and are more numerous in the blind-ended alveolar sacs. The alveolar membrane is the gas exchange surface, surrounded by a network of capillaries. Across the membrane oxygen is diffused into the capillaries and carbon dioxide released from the capillaries into the alveoli to be breathed out. Alveoli consist of an epithelial layer of simple squamous epithelium and an extracellular matrix surrounded by capillaries. The epithelial lining is part of the alveolar membrane, also known as the respiratory membrane.

Type I and type II pneumocytes are found in the alveolar wall. Alveolar macrophages are immune cells that move about in the alveolar lumen and in the connective tissue between them. Type I cells are squamous epithelial cells, thin and flat and form the structure of the alveoli. Type II cells (goblet cells) release pulmonary surfactant to lower surface tension.

A typical pair of human lungs contain about 300 million alveoli, producing 70 m² of surface area. Each alveolus is wrapped in a fine mesh of capillaries covering about 70% of its area. The diameter of a typical healthy alveolus is between 200 and 500 µm.

Inflammatory pulmonary diseases can be classified as follows (according to ICD-10 Chapter X: Diseases of the respiratory system (J00-J99), Version 2016, as of January 10th, 2020):

### a) Inflammation of the lower airways due to a bacterial, viral, fungal or parasitic infection

These diseases comprise, without being limiting, influenza due to identified avian influenza virus; influenza with pneumonia, influenza virus identified; influenza with other respiratory manifestations, influenza virus identified; influenza with other manifestations, influenza virus identified; influenza with pneumonia, virus not identified; influenza with other respiratory manifestations, virus not identified; influenza with other manifestations, virus not identified; adenoviral pneumonia; pneumonia due to *Streptococcus* pneumoniae; pneumonia due to *Haemophilus* influenzae; pneumonia due to *Klebsiella* pneumoniae; pneumonia due to *Pseudomonas;* pneumonia due to *Staphylococcus;* pneumonia due to *Streptococcus,* group B; pneumonia due to other streptococci; pneumonia due to *Escherichia coli;* pneumonia due to other aerobic Gram-negative bacteria; pneumonia due to *Mycoplasma pneumoniae;* other bacterial pneumonia; bacterial pneumonia, unspecified; chlamydial pneumonia; pneumonia due to other specified infectious organisms; pneumonia in bacterial diseases classified elsewhere; pneumonia in viral diseases classified elsewhere; pneumonia in mycoses; pneumonia in parasitic diseases; pneumonia in other diseases classified elsewhere; bronchopneumonia, unspecified; lobar pneumonia, unspecified; hypostatic pneumonia, unspecified; other pneumonia, organism unspecified; pneumonia, unspecified; acute bronchitis; acute bronchiolitis; unspecified acute lower respiratory infection.

### b) Chronic lower respiratory diseases

These diseases comprise, without being limiting, bronchitis, not specified as acute or chronic; simple and mucopurulent chronic bronchitis; chronic bronchitis; chronic tracheitis; chronic tracheobronchitis; emphysema; chronic obstructive pulmonary disease (COPD); asthma; status asthmaticus; bronchiectasis; sarcoidosis of the lung; pulmonary alveolar microlithiasis.

### c) Lung diseases due to an external agent

These diseases comprise, without being limiting, coalworker's pneumoconiosis; asbestosis; pneumoconiosis due to talc dust; silicosis; aluminosis of lung; bauxite fibrosis of lung; berylliosis; graphite fibrosis of lung; siderosis; stannosis; pneumoconiosis due to other specified inorganic dusts; unspecified pneumoconiosis; pneumoconiosis associated with tuberculosis; byssinosis; flax-dresser's disease; cannabinosis; airway disease due to other specific organic dusts; farmer's lung; bagassosis; bird fancier's lung; suberosis; maltworker's lung; mushroom-worker's lung; maple-bark-stripper's lung; air-conditioner and humidifier lung; hypersensitivity pneumonitis due to other organic dusts such as cheese-washer lung, coffee-worker lung, fishmeal-worker lung, furrier lung and sequiosis; hypersensitivity pneumonitis due to unspecified organic dust such as allergic alveolitis and hypersensitivity pneumonitis; respiratory conditions due to inhalation of chemicals, gases, fumes and vapors; pneumonitis due to solids and liquids; radiation pneumonitis; fibrosis of lung following radiation; acute drug-induced interstitial lung disorders; chronic drug-induced interstitial lung disorders; drug-induced interstitial lung disorders, unspecified; respiratory conditions due to other specified external agents; respiratory conditions due to unspecified external agent.

### d) Respiratory diseases principally affecting the interstitium

These diseases comprise, without being limiting, adult respiratory distress syndrome; pulmonary edema such as cardiogenic pulmonary edema, pulmonary permeability edema and high-altitude pulmonary edema; eosinophilic asthma; Loffler's pneumonia; tropical pulmonary eosinophilia; alveolar and parietoalveolar conditions; Hamman-Rich syndrome; pulmonary fibrosis; idiopathic pulmonary fibrosis; other specified interstitial pulmonary diseases; interstitial pulmonary disease, unspecified.

### e) Suppurative and/or necrotic conditions of lower respiratory tract

These diseases comprise, without being limiting, abscess of lung with pneumonia, pyothorax and empyema.

### f) Pleura diseases

These diseases comprise, without being limiting, pleurisy with effusion; pleural effusion in conditions classified elsewhere; pleural plaque; pneumothorax; chylothorax; fibrothorax; hemothorax; hemopneumothorax; hydrothorax; pleural condition, unspecified.

### g) Postprocedural or related lower respiratory diseases

These diseases comprise, without being limiting, acute pulmonary insufficiency following thoracic surgery; acute pulmonary insufficiency following nonthoracic surgery; chronic pulmonary insufficiency following surgery; host-versus-graft disease after lung transplantation; graft-versus-host disease after lung transplantation; chronic lung allograft dysfunction (CLAD), chronic lung allograft dysfunction - bronchiolitis obliterans syndrome (CLAD-BOS); lung ischemia reperfusion injury; primary graft dysfunction after lung transplantation; Mendelson's syndrome; other postprocedural respiratory disorders; postprocedural respiratory disorder, unspecified; respiratory failure, not elsewhere classified; diseases of bronchus, not elsewhere classified; pulmonary collapse; atelectasis; interstitial emphysema; mediastinal emphysema; compensatory emphysema; mediastinitis; disorders of diaphragm.

### h) Pulmonary diseases specific to the perinatal period

These diseases comprise, without being limiting, respiratory distress syndrome of newborn; transient tachypnoea of newborn; congenital pneumonia due to viral agent; congenital pneumonia due to *Chlamydia;* congenital pneumonia due to *Staphylococcus;* congenital pneumonia due to *Streptococcus,* group B; congenital pneumonia due to *Escherichia coli;* congenital pneumonia due to *Pseudomonas;* congenital pneumonia due to bacterial agents such as *Haemophilus influenzae, Klebsiella pneumoniae, Mycoplasma, Streptococcus,* except group B; congenital pneumonia due to other organisms; congenital pneumonia, unspecified; neonatal aspiration of meconium; interstitial emphysema originating in the perinatal period; pneumothorax originating in the perinatal period; pneumomediastinum originating in the perinatal period; other conditions related to interstitial emphysema originating in the perinatal period; pulmonary hemorrhage originating in the perinatal period; Wilson-Mikity syndrome; bronchopulmonary dysplasia originating in the perinatal period; unspecified chronic respiratory disease originating in the perinatal period.

### i) Trauma and injuries of the lower respiratory tract and/or the thorax

These conditions comprise, without being limiting, injury of pulmonary blood vessels; traumatic pneumothorax; traumatic hemothorax; traumatic hemopneumothorax; other injuries of lung; injury of bronchus; injury of pleura; injury of diaphragm; crushing injury of thorax and traumatic amputation of part of thorax.

### j) Malignant neoplasms of the lower respiratory tract

These diseases comprise, without being limiting, malignant neoplasm of bronchus and lung; lung cancer; non-small-cell lung cancer; adenocarcinoma; squamous-cell lung carcinoma; large-cell lung carcinoma; pulmonary enteric adenocarcinoma; bronchioloalveolar carcinoma; ovine pulmonary adenocarcinoma; small-cell lung cancer; bronchial leiomyoma; bronchial carcinoma; Pancoast tumor; pulmonary carcinoid tumor; pleuropulmonary blastoma; neuroendocrine tumors of the lung; lymphomas of the lung; lymphangiomatosis of the lung; sarcomas of the lung; alveolar soft part sarcoma; vascular tumors of the lung; mediastinal tumors; pleural tumors; metastasis to the lung.

### k) Inflammatory diseases of the pulmonary circulation

These diseases comprise, without being limiting, pulmonary embolism; primary pulmonary hypertension; pulmonary arterial hypertension; secondary pulmonary hypertension; pulmonary artery aneurysm.

Therefore, the present application refers to a human anti-inflammatory peptide according to the invention for use in the prophylaxis or treatment of inflammatory pulmonary diseases by inhalatory administration.

In detail, the present application refers to a human anti-inflammatory peptide according to the invention for use in the prophylaxis or treatment of inflammatory pulmonary diseases by inhalatory administration, wherein the inflammatory pulmonary disease is selected from the group comprising inflammations of the lower airways due to a bacterial, viral, fungal or parasitic infection, chronic lower respiratory diseases, lung diseases due to an external agent, respiratory diseases principally affecting the interstitium, suppurative and/or necrotic conditions of lower respiratory tract, pleura diseases, postprocedural or related lower respiratory diseases, pulmonary diseases specific to the perinatal period, trauma and injuries of the lower respiratory tract and/or the thorax, malignant neoplasms of the lower respiratory tract and inflammatory diseases of the pulmonary circulation.

In particular, the present application refers to a human anti-inflammatory peptide according to the invention for use in the prophylaxis or treatment of inflammatory pulmonary diseases by inhalatory administration, wherein the inflammatory pulmonary disease is an inflammation of the lower airways due to a bacterial, viral, fungal or parasitic infection.

In particular, the present application refers to a peptide according to the invention for use in the prophylaxis or treatment of inflammatory pulmonary diseases by inhalatory administration, wherein the inflammatory pulmonary disease is a chronic lower respiratory disease.

In particular, the present application refers to a peptide according to the invention for use in the prophylaxis or treatment of inflammatory pulmonary diseases by inhalatory administration, wherein the inflammatory pulmonary disease is a lung disease due to an external agent.

In particular, the present application refers to a peptide according to the invention for use in the prophylaxis or treatment of inflammatory pulmonary diseases by inhalatory administration, wherein the inflammatory pulmonary disease is a respiratory diseases principally affecting the interstitium.

In particular, the present application refers to a peptide according to the invention for use in the prophylaxis or treatment of inflammatory pulmonary diseases by inhalatory administration, wherein the inflammatory pulmonary disease is a suppurative and/or necrotic condition of the lower respiratory tract.

In particular, the present application refers to a peptide according to the invention for use in the prophylaxis or treatment of inflammatory pulmonary diseases by inhalatory administration, wherein the inflammatory pulmonary disease is a pleura disease.

In particular, the present application refers to a peptide according to the invention for use in the prophylaxis or treatment of inflammatory pulmonary diseases by inhalatory administration, wherein the inflammatory pulmonary disease is a postprocedural or related lower respiratory disease.

In particular, the present application refers to a peptide according to the invention for use in the prophylaxis or treatment of inflammatory pulmonary diseases by inhalatory administration, wherein the inflammatory pulmonary disease is a pulmonary disease specific to the perinatal period.

In particular, the present application refers to a peptide according to the invention for use in the prophylaxis or treatment of inflammatory pulmonary diseases by inhalatory administration, wherein the inflammatory pulmonary disease is a condition due to a trauma and/or injury of the lower respiratory tract and/or the thorax.

In particular, the present application refers to a peptide according to the invention for use in the prophylaxis or treatment of inflammatory pulmonary diseases by inhalatory administration, wherein the inflammatory pulmonary disease is a malignant neoplasm of the lower respiratory tract.

In particular, the present application refers to a peptide according to the invention for use in the prophylaxis or treatment of inflammatory pulmonary diseases by inhalatory administration, wherein the inflammatory pulmonary disease is an inflammatory disease of the pulmonary circulation.

In the scope of the present application COPD is of particular interest. COPD is a progressive development of airflow limitation that is not fully reversible. Most COPD patients suffer from three pathological conditions: Bronchitis, emphysema and mucus plugging. This disease is characterized by a slowly progressive and irreversible decrease in forced expiratory volume in the first second of expiration (FEV1), with relative preservation of forced vital capacity (FVC). In both asthma and COPD there is a significant but distinct remodeling of the airways. Most of the airflow obstruction is due to two major components, alveolar destruction (emphysema) and small airways obstruction (chronic obstructive bronchitis). COPD is mainly characterized by profound mucus cell hyperplasia. A primary characteristic of COPD is neutrophil infiltration into the patient's lungs. Elevated levels of proinflammatory cytokines, like TNF-alpha, and especially chemokines like interleukin-8 (IL-8) play a prominent role in the pathogenesis of COPD. Platelet thromboxane synthesis was found to be enhanced in COPD patients. Most of the tissue damage is caused by activation of neutrophils followed by their release of matrix metalloproteinases, and increased production of ROS and RNS.

Emphysema describes the destruction of the lung architecture with enlargement of the airspaces and loss of alveolar surface area. Lung damage is caused by weakening and breaking the air sacs within the lungs. Several adjacent alveoli may rupture, forming one large space instead of many small ones. Larger spaces can combine into an even bigger cavity, called a bulla. As a result, natural elasticity of the lung tissue gets lost, leading to overstretching and rupture, thus minimizing lung compliance. There is also less pull on the small bronchial tubes, which can cause their collapse and obstruct the airflow. Air not exhaled before the next breath cycle gets trapped in the lungs, leading to shortage of breath. The sheer effort it takes to force air out of the lungs upon exhaling is exhausting for the patients.

The most common symptoms of COPD include shortness of breath, chronic cough, chest tightness, greater effort to breathe, increased mucus production and frequent clearing of the throat. Patients become unable to perform their usual daily activities.

Long-term smoking is the most common cause of COPD, responsible for 80-90 % of all cases. Other risk factors are heredity, second-hand smoke, air pollution, and a history of frequent childhood respiratory infections. COPD is progressive and sometimes irreversible; there is currently no cure.

The clinical development of COPD is typically described in three stages:
Stage 1: Lung function (as measured by FEV1) is greater than or equal to 50% of predicted normal lung function. There is minimal impact on health-related quality of life. Symptoms may progress during this stage, and patients may begin to experience severe breathlessness, requiring evaluation by a pulmonologist.
Stage 2: FEV1 lung function is 35 to 49% of predicted normal lung function, and there is a significant impact on health-related quality of life.
Stage 3: FEV1 lung function is less than 35% of predicted normal lung function, and there is a profound impact on health-related quality of life.

Symptomatic pharmaceutical therapy includes the administration of bronchodilators, glucocorticoids and PDE4 inhibitors. Suitable bronchodilators are e.g. beta-2 adrenergic agonists such as the short-acting fenoterol and salbutamol as well as the long-acting salmeterol and formoterol, muscarinic anticholinergics such as ipratropium bromide and tiotropium bromide, and methylxanthines such as theophylline.

Suitable glucocorticoids include inhalatory glucocorticoids such as budesonide, beclometasone and fluticasone, orally administered glucocorticoids such as prednisolone and intravenously administered glucocorticoids such as prednisolone.

A suitable PDE (phosphodiesterase) 4 inhibitor is roflumilast.

Thus, the present application refers also to a peptide according to the invention for use in the prophylaxis or treatment of COPD by inhalatory administration.

In the scope of the present application also asthma is of particular interest. Asthma is a chronic inflammatory disease of the lower airways. It is mainly characterized by recurring symptoms such as reversible airflow obstruction and easily triggered bronchospasms. Symptoms include episodes of wheezing, coughing, chest tightness and dyspnea.

Asthma is thought to be caused by a combination of genetic and environmental factors. Environmental factors include exposure to air pollution and allergens. Other potential triggers may be iatrogenic.

Asthma is clinically classified into intermittent, mild persistent, moderate persistent and severe persistent, based on the frequency of symptoms. The most important parameters are FEV1 and peak expiratory flow rate.

Until now asthma cannot be cured. For long-term therapy, symptoms such as status asthmaticus can be prevented by avoiding triggers, such as allergens and irritants, and pharmacologically by the use of inhaled corticosteroids. Long-acting beta-2 agonists (LABA) or antileukotriene agents may be used additionally. The most common inhaled corticosteroids include beclomethasone, budesonide, fluticasone, mometasone and ciclesonide. Suitable LABA include salmeterol and formoterol. Leukotriene receptor antagonists such as montelukast, pranlukast and zafirlukast are orally administered. Suitable 5-lipooxygenase (5-LOX) inhibitors include meclofenamate sodium and zileuton. In severe stages of asthma intravenous corticosteroids such as prednisolone are recommended.

Acute asthma seizures (status asthmaticus) are best treated with inhaled short-acting beta-2 agonists such as salbutamol. Ipratropium bromide can be inhaled additionally. Intravenously, corticosteroids can be administered.

Inhalatory administration is commonly effected through metered-dose inhalers, respectively dry-powder inhalers.

Thus, the present application refers also to a peptide according to the invention in the prophylaxis or treatment of asthma by inhalatory administration.

In the scope of the present application also sarcoidosis of the lung is of particular interest. Sarcoidosis of the lung (interchangeably used herein: pulmonary sarcoidosis, PS) is characterized by abnormal collections of inflammatory cells that form lumps known as granulomas in the lung. The cause of sarcoidosis is unknown. The disease usually begins in the lungs, skin or lymph nodes, and can become manifest throughout the body. The most common symptom is a long-lasting fatigue, even if the disease activity has ceased. Overall malaise, shortness of breath, joint complaints, temperature increase, weight loss and skin complaints may be present. In general, the prognosis is good. Especially the acute form usually causes few problems, as the complaints will gradually decrease by themselves. If sarcoidosis is present in the heart, kidneys, liver and/or central nervous system, or extensively manifest in the lungs the outcome is less favorable. In general, sarcoidosis is classified in four stages determined by chest radiography. However, these stages do not correlate with the grade of severity. 1. bihilar lymphadenopathy (granulomas in the lymph nodes); 2. bihilar lymphadenopathy and reticulonodular infiltrates (granulomas in the lungs); 3. bilateral pulmonary infiltrates (granulomas in the lungs, but not in the lymph nodes); 4. fibrocystic sarcoidosis typically with upward hilar retraction, cystic and bullous changes (irreversible scarring in the lungs, i.e. pulmonary fibrosis). Stage 2 and 3 patients often display a chronic progressive disease course.

Symptomatic pharmaceutical therapy of sarcoidosis of the lung includes the administration of corticosteroids such as prednisone and prednisolone, immunosuppressive agents such as TNF-alpha inhibitors (etanercept, adalimumab, golimumab, infliximab), cyclophosphamide, cladribine, cyclosporine, chlorambucil and chloroquine, IL-23 inhibitors such as tildrakizumab and guselkumab, antimetabolites such as mycophenolic acid, leflunomide, azathioprine and methotrexate. In subtypes such as Löfgren's syndrome COX inhibitors such as acetylsalicylic acid, diclofenac or ibuprofen are used. All these active agents are administered systemically until now.

Thus, the present application refers also to a peptide according to the invention for use in the prophylaxis or treatment of sarcoidosis of the lung by inhalatory administration.

In the scope of the present application also cystic fibrosis is of particular interest. Cystic fibrosis is an inherited form of chronic bronchitis with mucus hypersecretion, generally accompanied by poor clearance of the airway secretions, obstruction of airflow and chronic bacterial infection of the airways, commonly by *Pseudomonas aeruginosa.* It is known that the sputum and bronchoalveolar lavage fluid from cystic fibrosis patients reduce the ability of neutrophils to kill these bacteria. Obstruction of the airways by such secretions can cause respiratory distress, and in some cases, can lead to respiratory failure and death. Further symptoms include sinus infections, poor growth, fatty stool, clubbing of the fingers and toes, and male infertility.

Cystic fibrosis is an autosomal-recessive hereditary disease caused by mutations in the gene for the cystic fibrosis transmembrane conductance regulator (CFTR) protein. CFTR is involved in the production of sweat, digestive fluids, and mucus. CFTR is a channel protein that controls the flow of H₂O and Cl⁻ ions in and out of cells inside the lungs. When the CFTR protein is working correctly, ions freely flow in and out of the cells. However, when the CFTR protein is malfunctioning, these ions cannot flow out of the cell due to a blocked channel. This causes cystic fibrosis, characterized by the buildup of thick mucus in the lungs.

No cure for cystic fibrosis is known. Intravenous, inhaled, and oral antibiotics are used to treat chronic and acute infections. Mechanical devices and inhalation medications are used to alter and clear the thickened mucus. These therapies, while effective, can be extremely time-consuming. Oxygen therapy at home is recommended in those with significant low oxygen levels. Inhaled antibiotics include e.g. levofloxacin, tobramycin, aztreonam and colistin. Orally administered antibiotics include e.g. ciprofloxacin and azithromycin. Mutation-specific CFTR potentiators are ivacaftor and tezacaftor.

Thus, the present application refers also to a peptide according to the invention for use in the prophylaxis or treatment of cystic fibrosis by inhalatory administration.

In the scope of the present application also bronchiectasis is of particular interest. Bronchiectasis is believed to be an idiopathic disease. Morphologically, it is characterized by a permanent enlargement of parts of the lower airways. As pathologic conditions, a.o. post-infection, immune deficiency, exaggerated immune response, congenital abnormalities, inflammatory pneumonitis, fibrosis and mechanical obstruction are discussed. Symptoms include chronic cough along with daily production of mucus. Thus, it resembles cystic fibrosis, but without the characteristic gene mutation. Pulmonary function testing results generally show airflow obstruction ranging from moderate to severe. Additional symptoms include dyspnea, coughing up blood, chest pain, hemoptysis, fatigue, and weight loss.

Treatment of bronchiectasis aims at controlling infections and bronchial secretions, relieving airway obstructions, removal of affected portions of lung by surgery or artery embolization. If indicated, antibiotics, in particular macrolide antibiotics are administered. Mucus overproduction can be addressed by mucolytics. Bronchodilators are used for facilitating breathing. Continuous inhaled corticosteroids help to some extent to reduce sputum production, to decrease airway constriction and to prevent disease progression.

Thus, the present application refers also to a peptide according to the invention in the prophylaxis or treatment of bronchiectasis by inhalatory administration.

In the scope of the present application also adult respiratory distress syndrome is of particular interest. Adult respiratory distress syndrome (ARDS) is a respiratory failure syndrome. It can have a variety of causes, such as pneumonia, trauma, severe burns, blood transfusion, aspiration, sepsis, pancreatitis or as a reaction on certain drugs. The gas exchange in the alveoli is seriously impaired due to injury of the alveolar endothelial cells, surfactant dysfunction, overshooting reaction of the immune system and coagulation disorders. A rapid migration of neutrophils and T lymphocytes into the affected lung tissue is observed. Acute symptoms include dyspnea, tachypnea and blue coloration of the skin. If the patient survives lung function is often permanently impaired. Acute treatment is mainly based on mechanical ventilation in intensive care units, and if indicated, the administration of antibiotics. Nitric oxide inhalation may help to improve oxygenation of the blood but has other drawbacks. Extracorporeal membrane oxygenation (ECMO) helps to increase the survival rate. However, pharmaceutical treatment, e.g. with corticosteroids, is controversially discussed.

Thus, the present application refers also to a peptide according to the invention for use in the prophylaxis or treatment of adult respiratory distress syndrome by inhalatory administration.

In the scope of the present application also pulmonary fibrosis is of particular interest. Herein scars are formed in the pulmonary tissues, leading to serious breathing problems.

Scar formation, respectively the accumulation of excess fibrous connective tissue leads to thickening of the walls, thus causing reduced oxygen supply in the blood. The consequence is a chronic progressive dyspnea. Pulmonary fibrosis is often secondary to other pulmonary diseases, e.g. in interstitial lung disorders, autoimmune diseases of the lung, inhalation of environmental and occupational pollutants, or certain infections. Otherwise, it is classified as idiopathic pulmonary fibrosis.

Pulmonary fibrosis involves gradual exchange of lung parenchyma with fibrotic tissue. The scar tissue causes an irreversible decrease in oxygen diffusion capacity, resulting in stiffness or decreased compliance of the lung. Pulmonary fibrosis is perpetuated by aberrant wound healing.

Up to now, there is no general pharmaceutical treatment of lung fibrosis. Some subtypes are responsive to corticosteroids such as prednisone, to anti-fibrotic agents such as pirfenidone and nintedanib, or to immunosuppressive agents, such as cyclophosphamide, azathioprine, methotrexate, penicillamine, and cyclosporine.

Thus, the present application refers also to a peptide according to the invention for use in the prophylaxis or treatment of pulmonary fibrosis, respectively idiopathic pulmonary fibrosis by inhalatory administration.

A typical inflammatory disease caused by an external agent is berylliosis (interchangeably herein, chronic beryllium disease, CBD). There is no cure for this occupational disease, only symptomatic treatment.

Prolonged exposure by inhalation may sensitize the lungs to beryllium, leading to the development of small inflammatory nodules, called granulomas. Typically, CBD granulomas are not characterized by necrosis and therefore not exhibiting a caseating appearance. Ultimately, this process leads to a decrease in pulmonary diffusion capacity. The typical symptoms are cough and dyspnea. Other symptoms include chest pain, joint aches, weight loss, and fever. The patient's T-cells become sensitized to beryllium. The pathologic immune response leads to an accumulation of CD4+ helper T-lymphocytes and macrophages in the lungs. There they aggregate together and form granulomas. Eventually, this leads to lung fibrosis. Treatment options include oxygen application and orally administered corticosteroids.

Thus, the present application refers also to a peptide according to the invention for use in the prophylaxis or treatment of berylliosis by inhalatory administration.

In the scope of the present application also chronic lung allograft dysfunction is of particular interest. Chronic lung allograft dysfunction (CLAD), respectively chronic lung allograft dysfunction - bronchiolitis obliterans syndrome (CLAD-BOS) is a major problem in the long-term management of lung transplant recipients. Both alloimmune-dependent factors (rejection) and alloimmune-independent factors contribute to the development of CLAD. It encompasses all forms of chronic pulmonary function decline, after eliminating known causes (persistent acute rejection, infection, anastomotic stricture, or disease recurrence, pleural disease, diaphragm dysfunction or native lung hyperinflation). Therefore, it is a heterogeneous entity in which two main phenotypes are currently identified: Bronchiolitis obliterans syndrome (BOS), defined by a persistent decline in FEV1, and an obstructive functional pattern.

No treatment is currently available to reverse CLAD after diagnosis. Pharmaceutical treatment of the symptoms is carried out with azithromycin (first line), alternatively montelukast. In therapy-resistant cases photopheresis is applied.

Thus, the present application refers also a peptide according to the invention for use in the prophylaxis or treatment of CLAD, respectively CLAD-BOS by inhalatory administration.

In the scope of the present application also pulmonary edema is of particular interest. Pulmonary edema may have different causes. Fluid accumulation occurs in the tissue and air spaces of the lungs, leading to impaired gas exchange and in the worst case to respiratory failure. Therapy for pulmonary edema focusses mainly on maintaining vital functions, e.g. by tracheal intubation and mechanical ventilation. Hypoxia symptoms can be addressed by oxygen supplementation

Cardiogenic pulmonary edema can be a result of congestive heart failure which is due to the heart's inability to pump the blood out of the pulmonary circulation at a sufficient rate resulting in elevation in wedge pressure and pulmonary edema. The underlying causes may be left ventricular failure, arrhythmias, or fluid overload, e.g., from kidney failure or intravenous therapy. It can be also caused by a hypertensive crisis, as the elevation in blood pressure and increased afterload on the left ventricle hinders forward flow and causes the elevation in wedge pressure and subsequent pulmonary edema. In acute cases, a loop diuretic such as furosemide is administered, often together with morphine to reduce respiratory distress. Both diuretic and morphine may have vasodilator effects, but also specific nitric oxide vasodilators such as intravenous glyceryl trinitrate or isosorbide dinitrate may be used.

Pulmonary permeability edema is characterized by reduced alveolar Na⁺ uptake capacity and capillary barrier dysfunction and is a potentially lethal complication, e.g. in listeriosis induced by listeriolysin. Apical Na⁺ uptake is mainly mediated by the epithelial sodium channel (ENaC) and initiates alveolar liquid clearance.

High-altitude pulmonary edema (HAPE) occurs in otherwise healthy people at altitudes typically above 2,500 meters and can be life-threatening. After a rapid gain in altitude, symptoms may include shortness of breath at rest, cough, weakness or decreased exercise performance, chest tightness or congestion, crackles or wheezing, central blue skin color, tachypnea and tachycardia. The lower air pressure at high altitudes leads to a decrease in partial pressure of arterial oxygen. Due to hypoxemia pulmonary hypertension secondary to hypoxic pulmonary vasoconstriction and increased capillary pressure develop. This leads to subsequent leakage of cells and proteins into the alveoli. Hypoxic pulmonary vasoconstriction occurs diffusely, leading to arterial vasoconstriction in all areas of the lung.

The first medical measure is a descent to a lower altitude as quickly as possible. Alternatively, oxygen supplementation for maintaining an S_{pO2} above 90% is possible.

Pharmaceutical prophylaxis of HAPE includes calcium channel blockers such as nifedipine, PDE5 inhibitors such as sildenafil and tadalafil and inhaled beta 2-agonists such as salmeterol.

A new pharmaceutical approach to enhance ENaC function is e.g. the peptide drug solnatide.

Thus, the present application refers also to a peptide according to the invention for use in the prophylaxis or treatment of pulmonary edema by inhalatory administration, in particular in the prophylaxis or treatment of cardiogenic pulmonary edema, pulmonary permeability edema and high-altitude pulmonary edema.

In the scope of the present application also lung ischemia reperfusion injury is of particular interest. In lung transplantation, organ ischemia and subsequent reperfusion is unavoidable and commonly leads to acute, sterile inflammation after transplant called ischemia-reperfusion (IR) injury. Severe IR injury leads to primary graft dysfunction (PGD), which is the major source of both short- and long-term morbidity and mortality after lung transplantation. Currently, there are no therapeutic agents clinically utilized to specifically prevent IR injury, and treatment strategies are limited to supportive care. If feasible, the donor lung, respectively the donor can be prophylactically treated with one of the peptides according to the invention.

Endothelial cell dysfunction and disruption of the endothelial barrier are hallmarks of lung IR injury. Depolarization of endothelial cell membranes induces ROS production and subsequent inflammation and leukocyte extravasation. Activation of NADPH oxidase (NOX2), induction of nitric oxide (NO) production, and activation of integrin αvβ5, promote vascular permeability via ROS/RNS production. Alveolar macrophages are activated. Elevated chemokine levels and adhesion molecule expression on endothelial cells and neutrophils lead to binding and infiltration of neutrophils, which can release cytokines, ROS and form neutrophil extracellular traps (NETs).

Recent prophylactic strategies before lung transplantation include administration to the organ recipient of anti-oxidants (free radical scavengers) or inhibitors of oxidant-producing enzymes (e.g. methylene blue or N-acetylcysteine), anti-inflammatory strategies using inhibitors of pro-inflammatory transcription factors or inflammatory mediators, ventilation with gaseous molecules such as carbon monoxide or inhaled anesthetic sevoflurane, growth factors or dietary supplements such as creatine, as well as cell-based therapies such as application of mesenchymal stem cells.

Thus, the present application refers also a peptide according to the invention for use in the prophylaxis or treatment of lung ischemia reperfusion injury by inhalatory administration.

In the scope of the present application also primary graft dysfunction after lung transplantation is of particular interest. Primary graft dysfunction (PGD) is a devastating form of acute lung injury that afflicts about 10% to 25% of patients in the first hours to days after lung transplantation. Clinically and pathologically it is a syndrome that mimics adult respiratory distress syndrome (ARDS) and carries a mortality of up to 50%. PGD can have different causes such as ischemia reperfusion injury described before, epithelial cell death, endothelial cell dysfunction, innate immune activation, oxidative stress, release of inflammatory cytokines and chemokines as well as iatrogenic factors such as mechanical ventilation and transfusion of blood components. Activation of the innate immune system activation has been demonstrated during the onset and spread of ischemia reperfusion injury. Herein PGD is associated with the innate immunity pathways of a Toll-like receptor-mediated injury.

Molecular markers of PGD include intracellular adhesion molecule-1, surfactant protein-1, plasminogen activator inhibitor, soluble receptor for advance glycation end products and protein C.

Approaches for avoiding PGD development include reperfusion optimization, regulation of prostaglandin levels, hemodynamic control, hormone replacement, ventilator management and donor lung preparation strategies. To decrease PGD incidence, strategies, such as using prostaglandins, nitric oxide, surfactant, adenosine or inhibition of pro-inflammatory mediators and/or elimination of free oxygen radicals, have been used. Furthermore, for the inhibition of neutrophils and neutrophil-borne mediators, free oxygen radicals, cytokines, proteases, lipid mediators, adhesion molecules and complement cascade inhibitors have been investigated. Inhaled nitric oxide may lower the pulmonary arterial pressure, without affecting the systemic blood pressure. As a last life-saving resort option, extracorporeal membrane oxygenation (ECMO) is used for correcting PGD-induced hypoxemia and by providing necessary gas exchange.

Thus, the present application refers also to a peptide according to the invention for use in the prophylaxis or treatment of primary graft dysfunction after lung transplantation by inhalatory administration.

For an effective prophylactic or therapeutic treatment of the aforementioned inflammatory pulmonary diseases the peptide according to the invention has to reach the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers described before. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable commercially available mesh nebulizers comprise, without being limiting, PARI eFlow®rapid, PARI LC STAR®, PARI Velox and PARI Velox Junior (PARI GmbH, Starnberg, Germany), Philips Respironics I-neb and Philips InnoSpire Go (Koninklijke Philips N.V., Eindhoven, Netherlands), VENTA-NEB®-ir, OPTI-NEB®, M-neb® dose⁺ mesh nebulizer inhalation MN-300/8, M-Neb Flow+ and M-neb® mesh nebulizer MN-300/X (NEBU-TEC, Eisenfeld, Germany), Hcmed Deepro HCM-86C and HCM860 (HCmed Innovations Co., Ltd, Taipei, Taiwan), OMRON MicroAir U22 and U100 (OMRON, Kyoto, Japan), Aerogen® Solo, Aerogen® Ultra and Aerogen® PRO (Aerogen, Galway, Ireland), KTMED NePlus NE-SM1 (KTMED Inc., Seoul, South Korea), Vectura Bayer Breelib™ (Bayer AG, Leverkusen, Germany), MPV Truma and MicroDrop® Smarty (MPV MEDICAL GmbH, Kirchheim, Germany), MOBI MESH (APEX Medical, New Taipei City, Taiwan), B.Well WN-114, TH-134 and TH-135 (B.Well Swiss AG, Widnau, Switzerland), Babybelle Asia BBU01 (Babybelle Asia Ltd., Hongkong), CA-MI Kiwi and others (CA-MI sri, Langhirano, Italy), Diagnosis PRO MESH (Diagnosis S.A., Bia 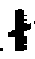 ystok, Poland), DIGI O₂ (DigiO₂ International Co., Ltd., New Taipei City, Taiwan), feellife AIR PLUS, AEROCENTRE+, AIR 360+, AIR GARDEN, AIRICU, AIR MASK, AIRGEL BOY, AIR ANGEL, AIRGEL GIRL and AIR PRO 4 (Feellife Health Inc., Shenzhen, China), Hannox MA-02 (Hannox International Corp., Taipei, Taiwan), Health and Life HL100 and HL100A (HEALTH & LIFE Co., Ltd., New Taipei City, Taiwan), Honsun NB-810B (Honsun Co., Ltd., Nantong, China), K-jump® KN-9100 (K-jump Health Co., Ltd., New Taipei City, Taiwan), microlife NEB-800 (Microlife AG, Widnau, Switzerland), OK Biotech Docspray (OK Biotech Co., Ltd., Hsinchu City, Taiwan), Prodigy Mini-Mist® (Prodigy Diabetes Care, LLC, Charlotte, USA), Quatek NM211, NE203, NE320 and NE403 (Big Eagle Holding Ltd., Taipei, Taiwan), Simzo NBM-1 and NBM-2 (Simzo Electronic Technology Ltd., Dongguan, China), Mexus® BBU01 and BBU02 (Tai Yu International Manufactory Ltd., Dongguan, China), TaiDoc TD-7001 (TaiDoc Technology Co., New Taipei City, Taiwan), Vibralung® and HIFLO Miniheart Circulaire II (Westmed Medical Group, Purchase, USA), KEJIAN (Xuzhou Kejian Hi-Tech Co., Ltd., Xuzhou, China), YM-252, P&S-T45 and P&S-360 (TEKCELEO, Valbonne, France), Maxwell YS-31 (Maxwell India, Jaipur, India), Kernmed® JLN-MB001 (Kernmed, Durmersheim, Germany).

Preferred are mesh nebulizers with a piezoelectric activation of the nebulization process, respectively vibrating mesh nebulizers.

Mesh nebulizers can be classified into two groups according to patient interaction: Continuous mode devices and trigger-activated devices. In continuous mode mesh nebulizers the nebulized aerosol is continuously released into the mouth piece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular trigger-activated vibrating mesh nebulizers.

Particularly preferred are trigger-activated mesh nebulizers with a piezoelectric activation of the nebulization process.

Preferred are the mesh nebulizer models PARI eFlow®rapid, Philips Respironics I-neb, Philips InnoSpire Go, M-neb® dose⁺ mesh nebulizer inhalation MN-300/8, Hcmed Deepro HCM-86C and HCM860, OMRON MicroAir U100, Aerogen® Solo, KTMED NePlus NE-SM1, Vectura Bayer Breelib™.

The most preferred vibrating mesh nebulizer models are high-end models such as PARI eFlow®rapid, PARI Velox, Philips Respironics I-neb, M-neb® dose⁺ mesh nebulizer inhalation MN-300/8, Vectura Bayer Breelib™.

Thus, the present application refers also to a peptide according to the invention for use in the prophylaxis or treatment of inflammatory pulmonary diseases by inhalatory administration, wherein a mesh nebulizer releases for the inhalatory administration an aerosol containing liquid droplets of a peptide according to the invention. This also holds true for the aforementioned subtypes of inflammatory pulmonary diseases as well as for the aforementioned single inflammatory pulmonary diseases, respectively.

The mean droplet size is usually characterized as MMAD (median mass aerodynamic diameter). The individual droplet size is referred to as MAD (mass aerodynamic diameter). This value indicates the diameter of the nebulized particles (droplets) at which 50% are smaller or larger, respectively. Particles with a MMAD > 10 µm normally don't reach the lower airways, they often get stuck in the throat. Particles with a MMAD > 5 µm and < 10 µm usually reach the bronchi but not the alveoli. Particles between 100 nm and 1 µm MMAD don't deposit in the alveoli and are exhaled immediately. Therefore, the optimal range is between 1 µm and 5 µm MMAD. Recent publications even favor a narrower range between 3.0 µm and 4.0 µm (cf. Amirav et al. (2010) J Allergy Clin Immunol 25: 1206-1211; Haidl et al. (2012) Pneumologie 66: 356-360).

Therefore, the MMAD of the nebulized human anti-inflammatory peptides according to the invention should be between 3.0 µm and 4.0 µm, preferably between 3.0 µm and 3.8 µm, more preferred between 3.0 and 3.7 µm, even more preferred between 3.0 µm and 3.6 µm, and most preferred between 3.0 µm and 3.5 µm.

A further commonly accepted quality parameter is the percentage of the particles in the generated aerosol with a diameter in the range of 1 µm to 5 µm (FPM; fine particle mass). FPM is a measure for the particle distribution. It is calculated by subtracting the percentage of the particles in the generated aerosol with a diameter in the range < 1 µm from the overall percentage of the particles in the generated aerosol with a diameter in the range < 5 µm (FPF; fine particle fraction).

Therefore, the FPM of the nebulized human anti-inflammatory peptides according to the invention should be at least 50%, preferably at least 55 % and most preferred at least 60%.

In order to test whether the combination of a mesh nebulizer and any of the nebulized peptides according to the invention is able to meet this objective the FPM in a thus generated aerosol was determined. As laid out in Examples 1 to 8, 10 ml of a 0.1 mg/ml physiological saline solution of one of the human anti-inflammatory peptides according to the invention was nebulized. It was surprisingly found that under these conditions 61.5 % - 83.5 % (FPM) of the particles were in the targeted range. This is a much more favorable particle size distribution percentage as found for many other comparable pharmaceutically active agents. This percentage may of course slightly vary according to the selected aqueous solution, temperature, mesh nebulizer model, selected piezoelectric excitation frequency, outlet geometry and dosage per application. Further, it was surprisingly found in the same experiments that the MMAD for this nebulization is 3.11 µm - 3.46 µm. Thus, this MMAD meets perfectly the desired range.

It is understood that the MMAD for this nebulization may slightly vary according to conditions such as ambient temperature, temperature of the pharmaceutical formulation to be nebulized, concentrations of the pharmaceutically active agent, optional choice of excipients etc.

The present application refers also to a human anti-inflammatory peptide according to the invention for use in the prophylaxis or treatment of inflammatory pulmonary diseases by inhalatory administration, wherein a mesh nebulizer releases for the inhalatory administration an aerosol containing liquid droplets of one of the peptides according to the invention, and at least 50% of the liquid droplets of the aerosol are in the size range of 1 µm to 5 µm in diameter.

In a more preferred embodiments at least 55% of the liquid droplets of the aerosol are in the size range of 1 µm to 5 µm in diameter.

In particularly preferred embodiments at least 60% of the liquid droplets of the aerosol are in the size range of 1 µm to 5 µm in diameter.

The present application refers also to a human anti-inflammatory peptide according to the invention for use in the prophylaxis or treatment of inflammatory pulmonary diseases by inhalatory administration, wherein a mesh nebulizer releases for the inhalatory administration an aerosol containing liquid droplets of said human anti-inflammatory peptide according to the invention, and the mass median aerodynamic diameter of these droplets is in the range of 3.0 µm to 4.0 µm.

In preferred embodiments the mass median aerodynamic diameter of these droplets is in the range of 3.0 µm to 3.8 µm.

In more preferred embodiments the mass median aerodynamic diameter of these droplets is in the range of 3.0 µm to 3.7 µm.

In even more preferred embodiments the mass median aerodynamic diameter of these droplets is in the range of 3.0 µm to 3.6 µm.

In particularly preferred embodiments the mass median aerodynamic diameter of these droplets is in the range of 3.0 µm to 3.5 µm.

Thus the present application refers to a human anti-inflammatory peptide according to the invention for use in the prophylaxis or treatment of inflammatory pulmonary diseases by inhalatory administration, wherein a mesh nebulizer releases for the inhalatory administration an aerosol containing liquid droplets of said human anti-inflammatory peptide according to the invention, and said aerosol is characterized in that the fine particle mass is at least 50% of the liquid droplets of the aerosol.

Thus the present application refers to a human anti-inflammatory peptide according to the invention for use in the prophylaxis or treatment of inflammatory pulmonary diseases by inhalatory administration, wherein a mesh nebulizer releases for the inhalatory administration an aerosol containing liquid droplets of said human anti-inflammatory peptide according to the invention, and said aerosol is characterized in that the median mass aerodynamic diameter of the liquid droplets of the aerosol is between 3.0 µm and 3.5 µm.

Thus the present application refers to a human anti-inflammatory peptide according to the invention for use in the prophylaxis or treatment of inflammatory pulmonary diseases by inhalatory administration, wherein a mesh nebulizer releases for the inhalatory administration an aerosol containing liquid droplets of said human anti-inflammatory peptide according to the invention, and said aerosol is characterized in that the fine particle fraction is at least 50% of the liquid droplets of the aerosol, and in that the median mass aerodynamic diameter of the liquid droplets of the aerosol is between 3.0 µm and 3.5 µm.

In another aspect, the present application refers to a human anti-inflammatory peptide according to the invention for use in an aerosol for inhalatory administration for the prophylaxis or treatment of inflammatory pulmonary diseases, wherein said aerosol is characterized in that the fine particle fraction is at least 50% of the liquid droplets of the aerosol, and in that the median mass aerodynamic diameter of the liquid droplets of the aerosol is between 3.0 µm and 3.5 µm.

For all aforementioned embodiments it is understood that said human anti-inflammatory peptide is selected from a group consisting of vasoactive intestinal peptide, C-type natriuretic peptide, B-type natriuretic peptide, pituitary adenylate cyclase-activating peptide, adrenomedullin, alpha-melanocyte stimulating hormone, relaxin and interferon gamma.

For all embodiments it is preferred that said mesh nebulizer is a vibrating mesh nebulizer.

This also holds true for the aforementioned subtypes of inflammatory pulmonary diseases as well as for the aforementioned single inflammatory pulmonary diseases, respectively.
In another aspect of the invention the present application refers to an aerosol produced by a mesh nebulizer, containing a peptide according to the invention in the range of 0.01% per weight to 10 % per weight,
an aqueous solution in the range of 70 % per weight to 99.99 % per weight, and optionally at least one pharmaceutically acceptable excipient in the range of 0 % per weight to 20 % per weight,
wherein said percentages add up to 100 %.

In another aspect of the invention the present application refers also to a pharmaceutical composition for use in the prophylaxis or treatment of inflammatory pulmonary diseases, comprising an aerosol produced by a nebulizer from an aqueous solution,
containing a peptide according to the invention in the range of 0.01% per weight to 10 % per weight,
an aqueous solution in the range of 70 % per weight to 99.99 % per weight, and optionally at least one pharmaceutically acceptable excipient in the range of 0 % per weight to 20 % per weight,
wherein said percentages add up to 100 %.

In another aspect of the invention the present application refers also to a method of treatment of inflammatory pulmonary diseases, comprising the steps of
a) providing an aerosol according to the invention by nebulization with a mesh nebulizer, and
b) administering a therapeutically effective amount of said aerosol to a patient in need thereof via a mouthpiece for inhalation fitting to said mesh nebulizer via self-inhalation by the patient.

The formulations of the peptides according to the invention may contain at least one pharmaceutically acceptable excipient.

The term "pharmaceutical excipients" refers to natural or synthetic compounds that are added to a pharmaceutical formulation alongside the pharmaceutical active agent. They may help to bulk up the formulation, to enhance the desired pharmacokinetic properties or the stability of the formulation, as well as being beneficial in the manufacturing process. Advantageous classes of excipients according to the invention include colorants, buffers, preservatives, antioxidants, pH regulators, solvents, isotonizing agents, opacifiers, aromatic and flavoring substances.

Colorants are excipients that bestow a colorization to the pharmaceutical formulation. These excipients can be food colorants. They can be adsorbed on a suitable adsorption means such as clay or aluminum oxide. A further advantage of a colorant is that it may visualize spilled aqueous solution on the nebulizer and/or the mouthpiece to facilitate cleaning. The amount of the colorant may vary between 0.01 and 10 % per weight of the pharmaceutical composition, preferred between 0.05 and 6 % per weight, more preferred between 0.1 and 4 % per weight, most preferred between 0.1 and 1 % per weight.

Suitable pharmaceutical colorants are for example curcumin, riboflavin, riboflavin-5'-phosphate, tartrazine, alkannin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulfite caramel, ammonia caramel, sulfite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Moreover, buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)methylaminol] propane), ethylene diamine, ACES (2-[(amino-2-oxoethyl)amino]ethanesulfonic acid), imidazole, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholino-propanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)glycine and N-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, N,N,N-trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid and/or solid dosage forms can be used on demand. They may be selected from the group comprising, but not limited to, sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

Suitable solvents may be selected from the group comprising, but not limited to, water, carbonated water, water for injection, water with isotonizing agents, saline, isotonic saline, alcohols, particularly ethyl and n-butyl alcohol, and mixtures thereof.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

The addition of a sufficient amount of antioxidants is particularly preferable for liquid dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluene, fumaric acid or propyl gallate. Preferred is the use of alpha-tocopherol and ascorbyl palmitate.

Suitable pH-regulators for liquid dosage forms are e.g. sodium hydroxide, hydrochloric acid, buffer substances such as sodium dihydrogen phosphate or disodium hydrogenphosphate.

Suitable aromatic and flavoring substances comprise above all essential oils that can be used for this purpose. In general, this term refers to volatile extracts from plants or parts of plants with the respective characteristic smell. They can be extracted from plants or parts of plants by steam distillation.

Suitable examples are: Essential oils, respectively aromatic substances from sage, cloves, chamomile, anise, star anise, thyme, tea tree, peppermint, mint oil, menthol, cineol, borneol, zingerol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor.

These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Opacifiers are substances that render the liquid dosage for, opaque, if desired. They must have a refractive index substantially different from the solvent, in most cases here water. At the same time they should be inert to the other components of the composition. Suitable examples include titanium dioxide, talc, calcium carbonate, behenic acid, cetyl alcohol, or mixtures thereof.

According to the invention all of the aforementioned excipients and classes of excipients can be used without limitation alone or in any conceivable combination thereof, as long as the inventive use is not thwarted, toxic actions may occur or the respective national legislations are infracted.

In another aspect of the invention the present application refers also to a method for producing an aerosol according to the invention, comprising the following steps:
a) filling 0.1 ml to 10 ml of an aqueous solution containing a peptide according to the invention and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

The vibration frequency of vibrating mesh nebulizers is normally in the range of 80 kHz to 200 kHz. Therefore the present application refers to the use according to the invention, wherein the vibration frequency of the vibrating mesh nebulizer is in the range of 80 kHz to 200 kHz, preferred 90 kHz to 180 kHz, more preferred 100 kHz to 160 kHz, most preferred 105 kHz to 130 kHz (cf. Chen, *The Aerosol Society*: **DDL2019;** Gardenshire et al. (2017) A Guide to Aerosol Delivery Devices for Respiratory Therapists, 4th ed.).

Thus, the aforementioned method is also disclosed with said vibration frequency ranges.

As can be seen in the aerosol analyses shown in the examples, the method of the invention proved to be particularly effective in nebulizing a high percentage of the pharmaceutically active agent from the provided aqueous solution. Therefore, there is a relatively small loss of the pharmaceutically active agent during the nebulization step.

As can be seen in the aerosol analysis and the respective experimental settings of Examples 1 - 8, the method of the invention proved to be particularly effective in nebulizing a high percentage of the pharmaceutically active agent from the provided aqueous solution during a short time. This is an important feature for patient compliance. A considerable percentage of the patient population finds the inhalatory process to be uncomfortable, weary and physically demanding. On the other hand, the patient's active cooperation is essential for an effective and targeted inhalatory application. Therefore, it is desirable that a therapeutically sufficient amount is applied during a period of time as short as possible. Surprisingly, it showed that during a three minutes time span 95 % of the substance provided in the aqueous solution could be nebulized. This is an ideal time span for a high patient compliance.

Therefore, the method according to the invention is thus characterized in that at least 80 % of the generated aerosol are produced during three minutes after starting nebulization in the mesh nebulizer, preferred at least 85 % and most preferred at least 90 %.

While the pharmaceutically active agent is usually provided in a single dosage container for every nebulization procedure the nebulizer and/or the mouthpiece can be used over a certain period of time and have to be replaced at certain intervals. A cleaning of the nebulizer and the mouthpiece is recommended by default after each nebulization. But herein patient compliance cannot be reasonably taken for granted. But even after a meticulous cleaning there are always some deposits of the aerosol in the nebulization chamber, the outlet and/or the mouthpiece. As the aerosol is produced from an aqueous solution these depositions bear the risk of producing a bioburden of bacteria that might contaminate the inhaled aerosol. Deposits may also plug holes in the mesh membrane of the mesh nebulizer. In general, the nebulizer and/or the mouthpiece should be exchanged every one or two weeks. Therefore, it is convenient to offer the medication and the nebulizer as a combined product.

Thus, in another aspect of the invention the present application refers also to a kit comprising a mesh nebulizer and a pharmaceutically acceptable container with an aqueous solution containing a peptide according to the invention and optionally at least one pharmaceutically acceptable excipient.

In an alternative kit the peptide according to the invention is not provided in form of an aqueous solution but in two separated containers, one for a solid form for the active agent and the other for an aqueous solution. The final aqueous solution is freshly prepared by solving the active agent in the final solution. Thereupon the final aqueous solution is filled into the nebulization chamber of the mesh nebulizer. These two containers can be completely separated containers, e.g. two vials, or e.g. a dual-chamber vial. For solving the active agent e.g. a membrane between the two chambers is perforated to allow for mixing of the content of both chambers.

Thus, the present application discloses also a kit, comprising a mesh nebulizer, a first pharmaceutically acceptable container with water for injection or physiological saline solution and a second pharmaceutically acceptable container with a solid form of a peptide according to the invention, wherein optionally at least one pharmaceutically acceptable excipient is contained in the first pharmaceutically acceptable container and/or the second pharmaceutically acceptable container.

The aerosol generated by the method according to the invention is administered, respectively self-administered by means of a mouthpiece. Optionally, such a mouthpiece can be additionally included in the beforementioned kits.

A common way to transfer the provided aqueous solution or final aqueous solution into the nebulization chamber of the mesh nebulizer by means of a syringe equipped with an injection needle. First, the aqueous solution is drawn up into the syringe and then injected into the nebulization chamber. Optionally, such a syringe and/or injection needle can be additionally included in the beforementioned kits. Without being limiting, typical syringes made of polyethylene, polypropylene or cyclic olefin co-polymers can be used, and a typical gauge for a stainless steel injection needle would be in the range of 14 to 27.

### EXAMPLES

All tested peptides (incl. Aviptadil) were purchased from Bachem AG, Bubendorf, Switzerland. No peptide extracted from an organism was tested.

### Example 1:

The particle size distribution (FPM) and the mass median aerodynamic diameter (MMAD) of an aerosol generated from an aqueous solution containing vasoactive intestinal peptide was analyzed.

Vasoactive intestinal peptide was solved in physiological saline solution (double distilled water containing 0.9 % NaCI). The final concentration solution was 0.1 mg/ml vasoactive intestinal peptide.

The experimental procedure was carried out pursuant to Ph. Eur. 2.9.44 (Preparations for Nebulisation: Characterisation). A cascade impactor (Next Generation Impactor (NGI), Copley Scientific Ltd., Nottingham, UK) was used to determine the respective percentage for each fraction of the nebulized particles.

The NGI comprises the following features:
1) Designed and accepted by pharma and biotech industry for inhaler testing;
2) Meets and exceeds all European and US Pharmacopoeia specifications;
3) Particle size range between 0.24 - 11.7 µm (dependent on flow rate);
4) 7 stages, 5 with cut-offs between 0.54 - 6.12 µm at flow rates from 30 to 100 l/min;
5) Calibrated flow rate range from 30 -100 l/min;
6) Additional calibration at 15 l/min for nebulizer applications;
7) Supplied with full stage mensuration report (system suitability);
8) Low inter-stage wall losses for good drug recovery (mass balance);
9) Electrically conductive and unaffected by static;
The NGI cascade impactor itself comprises three main parts:
a) The cup tray containing the eight collection cups used to collect the samples prior to analysis
b) The bottom frame used to support the cup tray
c) The lid containing the inter-stage passageways and the seal body which holds the nozzles in place.

The nebulization of this aqueous solution of vasoactive intestinal peptide was carried out by means of a vibrating mesh nebulizer (M-neb-dose+, NEBU-TEC, Eisenfeld, Germany). The thus generated aerosol was delivered to the cascade impactor by means of a mouthpiece (SK-211, NEBU-TEC, Eisenfeld, Germany).

The nebulization chamber (yellow stamp, 250 µl liquid discharge) was filled. The puff profile configured at the breath simulator was chosen in such a way that a correct simulation of inhalation by means of the nebulizer was ensured. The corresponding time was recorded.

The quantification of nebulized vasoactive intestinal peptide per cascade was carried out after extraction from the cascade pans (stage 1 - 8) by means of HPLC with reference to an external standard calibration curve (range 0 - 200 µg/ml; correlation coefficient: 0.9999).

Single values obtained for each cascade were added. No residual amounts vasoactive intestinal peptide could be found in the mouthpiece. MMAD, FPF and FPM were calculated from the obtained values:

| | |
|---|---|
| released dosage | 0.80 mg |
| MMAD | 3.40 µm |
| FPF | 79.1 % |
| FPF | 0.63 mg |
| FPM | 75.1 % |
| FPM | 0.60 mg |

The aerosol can be further characterized as follows:
- The released dosage at the mouthpiece of 0.80 mg corresponds to 80 % of the nominal value
- The time of aerosol release was 3 min (corresponds to 30 strokes / breaths)
- The ratio of particles with a MMAD < 1 µm to particles with a MMAD > 1 µm is 5 : 95.

### Example 2:

The particle size distribution (FPM) and the mass median aerodynamic diameter (MMAD) of an aerosol generated from an aqueous solution containing C-type natriuretic peptide was analyzed. The procedure was the same as in Example 1.

C-type natriuretic peptide was solved in physiological saline solution (double distilled water containing 0.9 % NaCI). The final concentration solution was 0.1 mg/ml C-type natriuretic peptide.

The following results were found:

| | |
|---|---|
| released dosage | 0.77 mg |
| MMAD | 3.37 µm |
| FPF | 87.9 % |
| FPF | 0.68 mg |
| FPM | 83.5 % |
| FPM | 0.64 mg |

The aerosol can be further characterized as follows:
- The released dosage at the mouthpiece of 0.77 mg corresponds to 77 % of the nominal value
- The time of aerosol release was 3 min (corresponds to 30 strokes / breaths)
- The ratio of particles with a MMAD < 1 µm to particles with a MMAD > 1 µm is 5 : 95.

### Example 3:

The particle size distribution (FPM) and the mass median aerodynamic diameter (MMAD) of an aerosol generated from an aqueous solution containing B-type natriuretic peptide was analyzed. The procedure was the same as in Example 1.

B-type natriuretic peptide was solved in physiological saline solution (double distilled water containing 0.9 % NaCI). The final concentration solution was 0.1 mg/ml B-type natriuretic peptide.

The following results were found:

| | |
|---|---|
| released dosage | 0.69 mg |
| MMAD | 3.46 µm |
| FPF | 65.5 % |
| FPF | 0.45 mg |
| FPM | 62.2 % |
| FPM | 0.43 mg |

The aerosol can be further characterized as follows:
- The released dosage at the mouthpiece of 0.69 mg corresponds to 69 % of the nominal value
- The time of aerosol release was 3 min (corresponds to 30 strokes / breaths)
- The ratio of particles with a MMAD < 1 µm to particles with a MMAD > 1 µm is 5 : 95.

### Example 4:

The particle size distribution (FPM) and the mass median aerodynamic diameter (MMAD) of an aerosol generated from an aqueous solution containing Pituitary Adenylate Cyclase-activating Peptide (PACAP-38) was analyzed. The procedure was the same as in Example 1.

Pituitary Adenylate Cyclase-activating Peptide was solved in physiological saline solution (double distilled water containing 0.9 % NaCl). The final concentration solution was 0.1 mg/ml Pituitary Adenylate Cyclase-activating Peptide.

The following results were found:

| | |
|---|---|
| released dosage | 0.58 mg |
| MMAD | 3.25 µm |
| FPF | 87.7 % |
| FPF | 0.51 mg |
| FPM | 82.4 % |
| FPM | 0.48 mg |

The aerosol can be further characterized as follows:
- The released dosage at the mouthpiece of 0.58 mg corresponds to 58 % of the nominal value
- The time of aerosol release was 3 min (corresponds to 30 strokes / breaths)
- The ratio of particles with a MMAD < 1 µm to particles with a MMAD > 1 µm is 6: 94.

### Example 5:

The particle size distribution (FPM) and the mass median aerodynamic diameter (MMAD) of an aerosol generated from an aqueous solution containing adrenomedullin was analyzed. The procedure was the same as in Example 1.

Adrenomedullin was solved in physiological saline solution (double distilled water containing 0.9 % NaCl). The final concentration solution was 0.1 mg/ml adrenomedullin.

The following results were found:

| | |
|---|---|
| released dosage | 0.53 mg |
| MMAD | 3.41 µm |
| FPF | 64.7 % |
| FPF | 0.34 mg |
| FPM | 61.5 % |
| FPM | 0.33 mg |

The aerosol can be further characterized as follows:
- The released dosage at the mouthpiece of 0.53 mg corresponds to 53 % of the nominal value
- The time of aerosol release was 3 min (corresponds to 30 strokes / breaths)
- The ratio of particles with a MMAD < 1 µm to particles with a MMAD > 1 µm is 5 : 95.

### Example 6:

The particle size distribution (FPM) and the mass median aerodynamic diameter (MMAD) of an aerosol generated from an aqueous solution containing α-melanocyte stimulating hormone was analyzed. The procedure was the same as in Example 1.

α-Melanocyte stimulating hormone was solved in physiological saline solution (double distilled water containing 0.9 % NaCI). The final concentration solution was 0.1 mg/ml α-melanocyte stimulating hormone.

The following results were found:

| | |
|---|---|
| released dosage | 0.45 mg |
| MMAD | 3.11 µm |
| FPF | 86.7 % |
| FPF | 0.39 mg |
| FPM | 80.6 % |
| FPM | 0.36 mg |

The aerosol can be further characterized as follows:
- The released dosage at the mouthpiece of 0.45 mg corresponds to 45 % of the nominal value
- The time of aerosol release was 3 min (corresponds to 30 strokes / breaths)
- The ratio of particles with a MMAD < 1 µm to particles with a MMAD > 1 µm is 7 : 93.

### Example 7:

The particle size distribution (FPM) and the mass median aerodynamic diameter (MMAD) of an aerosol generated from an aqueous solution containing relaxin-3 was analyzed. The procedure was the same as in Example 1.

Relaxin-3 was solved in physiological saline solution (double distilled water containing 0.9 % NaCI). The final concentration solution was 0.1 mg/ml relaxin-3.

The following results were found:

| | |
|---|---|
| released dosage | 0.49 mg |
| MMAD | 3.36 µm |
| FPF | 80.7 % |
| FPF | 0.40 mg |
| FPM | 76.7 % |
| FPM | 0.38 mg |

The aerosol can be further characterized as follows:
- The released dosage at the mouthpiece of 0.49 mg corresponds to 49 % of the nominal value
- The time of aerosol release was 3 min (corresponds to 30 strokes / breaths)
- The ratio of particles with a MMAD < 1 µm to particles with a MMAD > 1 µm is 5 : 95.

### Example 8:

The particle size distribution (FPM) and the mass median aerodynamic diameter (MMAD) of an aerosol generated from an aqueous solution containing interferon gamma was analyzed. The procedure was the same as in Example 1.

Interferon gamma was solved in physiological saline solution (double distilled water containing 0.9 % NaCl). The final concentration solution was 0.1 mg/ml interferon gamma.

The following results were found:

| | |
|---|---|
| released dosage | 0.45 mg |
| MMAD | 3.46 µm |
| FPF | 64.8 % |
| FPF | 0.29 mg |
| FPM | 62.9 % |
| FPM | 0.28 mg |

The aerosol can be further characterized as follows:
- The released dosage at the mouthpiece of 0.45 mg corresponds to 95 % of the nominal value
- The time of aerosol release was 3 min (corresponds to 30 strokes / breaths)
- The ratio of particles with a MMAD < 1 µm to particles with a MMAD > 1 µm is 3 : 97.

### Example 9:

For comparative purposes, the particle size distribution (FPM) and the mass median aerodynamic diameter (MMAD) of an aerosol generated from an aqueous solution containing colistin was analyzed (not part of the invention). The procedure was the same as in Example 1.

Colistin (Polymyxin E) is a polypeptide antibiotic from the polymyxin class. It is produced by certain strains of the bacteria *Paenibacillus polymyxa.* As a drug, colistin is administered as colistin sulfate or colistimethate sodium. Topical, peroral, intravenous and inhaled dosage forms are available.

Colistin is effective in treating infections caused by *Pseudomonas aeruginosa, Escherichia coli,* and *Klebsiella pneumoniae* (Falagas et al. (2008) Expert Review of Anti-infective Therapy 6: 593-600). In combination with other drugs colistin is used to attack biofilm infection in the lung of cystic fibrosis patients. Biofilms have a low oxygen environment below the surface where bacteria are metabolically inactive while colistin is highly effective in this environment.

Colistin was solved in physiological saline solution (double distilled water containing 0.9 % NaCI). The final concentration solution was 0.1 mg/ml colistin.

The following results were found:

| | |
|---|---|
| released dosage | 0.75 mg |
| MMAD | 3.61 µm |
| FPF | 72.8 % |
| FPF | 0.55 mg |
| FPM | 69.7 % |
| FPM | 0.52 mg |

The aerosol can be further characterized as follows:
- The released dosage at the mouthpiece of 0.75 mg corresponds to 75 % of the nominal value
- The time of aerosol release was 3 min (corresponds to 30 strokes / breaths)
- The ratio of particles with a MMAD < 1 µm to particles with a MMAD > 1 µm is 4 : 96.

### Example 10:

For comparative purposes, the particle size distribution (FPM) and the mass median aerodynamic diameter (MMAD) of an aerosol generated from an aqueous solution containing dornase alfa was analyzed (not part of the invention). The procedure was the same as in Example 1.

Dornase alfa is a recombinant human deoxyribonuclease I (rhDNase), a polypeptide which selectively cleaves DNA. Dornase alfa hydrolyzes the DNA present in sputum/mucus of cystic fibrosis patients and thus reduces the viscosity of fluids in the lungs, promoting improved clearance of secretions. This polypeptide therapeutic agent is produced in Chinese Hamster Ovary (CHO) cells.

Dornase alfa was solved in physiological saline solution (double distilled water containing 0.9 % NaCI). The final concentration solution was 0.1 mg/ml dornase alfa.

The following results were found:

| | |
|---|---|
| released dosage | 0.65 mg |
| MMAD | 3.72 µm |
| FPF | 70.0 % |
| FPF | 0.46 mg |
| FPM | 66.3 % |
| FPM | 0.43 mg |

The aerosol can be further characterized as follows:
- The released dosage at the mouthpiece of 0.65 mg corresponds to 65 % of the nominal value
- The time of aerosol release was 3 min (corresponds to 30 strokes / breaths)
- The ratio of particles with a MMAD < 1 µm to particles with a MMAD > 1 µm is 5 : 95.

### Example 11:

A patient (female, 45 years) with severe pulmonary arterial hypertension (PAH), associated with collagen vascular disease, presented with signs of right ventricular decompensation, with an initial pulmonary vascular resistance (PVR) of 1413 dyn s cm⁻⁵. The patient was treated before with bosentan for 3 months. This therapy was discontinued due to increased liver enzymes. PAH-specific medication was changed to triple therapy comprising inhaled iloprost and systemic sildenafil and ambrisentan. The patient developed peripheral edema, right ventricular hypertrophy, right ventricular systolic pressure (RVSP) 97 mm Hg, TAPSE (tricuspid annular plane systolic excursion) 17 mm, 6 min walking test 290 m. A repeated prostanoid therapy was refused by the patient. Due to the lack of other treatment options, treatment with inhaled Aviptadil (vasoactive intestinal peptide) was initiated at a dose of 4 x 140 µg/ml per day (560 µg/day) with overnight break, droplet characteristics of a MMAD of 3.4 µm per emitted particle (nebulizer: M-neb-dose+, NEBU-TEC, Eisenfeld, Germany). 90% of the dose were delivered at the mouthpiece (SK-211, NEBU-TEC, Eisenfeld, Germany). Inhalation time per treatment was 12 minutes. A gradual improvement of the patient was achieved with cardiac recompensation. 6 months later the 6 min walking test was increased to 340 m, 12 months later to 410 m. 3 years later an ongoing improvement was noted, with 6 min walking distances between 430 - 475 m and still persistent PAH, but good right ventricular function of TAPSE 22 mm. This dramatic improvement of the patient over time is attributed to the effect of inhaled vasoactive intestinal peptide.

### Example 12:

A patient (male, 65 years) with severe sarcoidosis of the lung indicated for therapeutic treatment suffered from unproductive chronic cough, dyspnea on exertion and chronic fatigue. The patient did not tolerate treatment with corticosteroids and severe immunosuppressants, and due to the lack of other available treatment options, the patient qualified for the Early Access Program treatment with inhaled Aviptadil (vasoactive intestinal peptide) (vibrating mesh nebulizer: M-neb-dose+, NEBU-TEC, Eisenfeld, Germany; mouthpiece: SK-211, NEBU-TEC, Eisenfeld, Germany). Therapy was initiated at a dose of 4 x 70 µg/ml per day dosage (280 µg per day with overnight break; nebulizer: M-neb-dose+, NEBU-TEC, Eisenfeld, Germany; mouthpiece; SK-211, NEBU-TEC, Eisenfeld, Germany). Therapeutic benefit was measured by the newly established and well validated quality of life questionnaire for sarcoidosis, the King's College Sarcoidosis Questionnaire (KSQ; Patel et al. (2013) Thorax 68: 57-65). Therein an increase in score by 5 over time indicates significant clinical improvement. The patient started treatment at a score of 66, improved after 3 months of treatment to score 71, and after 6 months of treatment to score 77, which is 11 points higher than at beginning of treatment with vasoactive intestinal peptide.

### Example 13:

A patient (male, 65 years) who was continuously exposed to beryllium at his workplace developed Chronic Beryllium Disease (CBD). This was diagnosed by tests performed on peripheral blood and bronchoalveolar lavage (BAL) mononuclear cells. The 14se tests showed a dose-dependent proliferation of specific T-cell clones in response to *in vitro* beryllium exposure. Cells from healthy control subjects or patients with other granulomatous disorders do not change in their proliferation rate when exposed to beryllium. A test called beryllium lymphocyte proliferation test (BeLPT) confirmed the CBD diagnosis. Due to the lack of available treatment options for CBD, the patient qualified for the Early Access Program treatment with inhaled Aviptadil (vasoactive intestinal peptide). Therapy was initiated at a dose of 4 x 70 µg/ml per day dosage (280 µg per day with overnight break; nebulizer: M-neb-dose+, NEBU-TEC, Eisenfeld, Germany; mouthpiece; SK-211, NEBU-TEC, Eisenfeld, Germany). After 6 months of treatment, the proliferation rate of PBMCs stimulated with beryllium (0.1 mM) from the patient was inhibited by 100% by vasoactive intestinal peptide. The production of the cytokines typical for the onset of the disease (TNF-alpha, IL-17) was significantly diminished in the patient.

### Example 14:

A patient (male, 70 years) was diagnosed with severe Chronic Obstructive Pulmonary Disease (COPD), GOLD (Global Initiative for Chronic Obstructive Lung Disease scoring system) stage 4D (FEV1 < 30 % of predicted value and more than two exacerbations per year with one exacerbation which required hospitalization) suffering from pulmonary emphysema and pulmonary cachexia, unproductive chronic cough, dyspnea, chronic fatigue, and restless sleep.

The patient underwent therapeutic treatment with tiotropium bromide, olodaterol, salbutamol and ipratropium bromide for more than three years without disease stabilization or improvement. Due to the lack of additional available treatment options for COPD, the patient qualified for the Early Access Program treatment with inhaled Aviptadil (vasoactive intestinal peptide). Therapy was initiated at a dose of 2 x 140 µg/ml per day dosage (280 µg per day with overnight break; nebulizer: M-neb-dose+, NEBU-TEC, Eisenfeld, Germany; mouthpiece; SK-211, NEBU-TEC, Eisenfeld, Germany).

The patient monitored the disease progress by using the COPD Assessment Test (CAT - German version; https://www.catestonline.org/patient-site-test-page-german-germany.html, as of January 22nd, 2020). After three months of treatment, the CAT score improved from 31 to 25. After five years of suffering, the patient was finally able to sleep well, and stop coughing, while his mood and social life significantly improved.

**List of abbreviations:**

| | |
|---|---|
| ACES | 2-[(amino-2-oxoethyl)amino]ethanesulfonic acid |
| ADM | adrenomedullin |
| AM | adrenomedullin |
| API | active pharmaceutical ingredient |
| ARDS | adult respiratory distress syndrome |
| BAL | bronchoalveolar lavage |
| BeLPT | beryllium lymphocyte proliferation test |
| BES | N,N-bis-(2-hydroxyethyl)-2-aminoethanesulfonic acid |
| BNP | brain natriuretic peptide |
| BNPT | NT-proBNP |
| BOS | bronchiolitis obliterans syndrome |
| cAMP | adenosine 3,5-cyclic monophosphate |
| CAT | COPD Assessment Test |
| CBD | chronic beryllium disease |
| CBP | CREB-binding protein |
| CFTR | cystic fibrosis transmembrane conductance regulator |
| cGMP | cyclic guanosine monophosphate |
| CGRP | calcitonin gene-related peptide |
| CHO | Chinese hamster ovary |
| CIRS | chronic inflammatory response syndrome |
| CLAD | chronic lung allograft dysfunction |
| CLAD-BOS | chronic lung allograft dysfunction - bronchiolitis obliterans syndrome |
| CNP | C-type natriuretic peptide |
| COPD | chronic obstructive pulmonary disease |
| COX | cyclooxygenase |
| CREB | cAMP-responsive-element-binding protein |
| CRLR | calcitonin receptor-like receptor |
| DNA | deoxyribonucleic acid |
| DPI | dry powder inhaler |
| DPLD | diffuse parenchymal lung diseases |
| ECMO | extracorporeal membrane oxygenation |
| EEPS | 4-(2-hydroxyethyl)piperazine-1-propanesulfonic acid |
| ENaC | epithelial sodium channel |
| FEV1 | forced expiratory volume in the first second of expiration |
| FPF | fine particle fraction |
| FPM | fine particle mass |
| FVC | forced vital capacity |
| GOLD | Global Initiative for Chronic Obstructive Lung Disease scoring system |
| HAPE | high-altitude pulmonary edema |
| HEPES | N-2-hydroxyethylpiperazine-N'-2-ethanesulfonic acid |
| ICD-10 | International Statistical Classification of Diseases and Related Health Problems, 10th revision |
| IFN-γ | interferon gamma |
| IκK | IkappaB kinase |
| IL | interleukin |
| IPF | idiopathic pulmonary fibrosis |
| IR | ischemia-reperfusion |
| JNK | JUN kinase |
| KSQ | King's College Sarcoidosis Questionnaire |
| LABA | long-acting beta-2 agonist |
| 5-LOX | 5-lipoxygenase |
| m² | square meter |
| MAD | mass aerodynamic diameter |
| MDI | metered-dose inhaler |
| MEKK1 | MAPK/ERK kinase kinase 1 |
| MES | 2-(N-morpholino)ethanesulfonic acid |
| MMAD | median mass aerodynamic diameter |
| MOPS | 3-(N-morphino) propanesulfonic acid |
| α-MSH | α-melanocyte stimulating hormone |
| µm | micrometer |
| NETs | neutrophil extracellular traps |
| NF-κB | nuclear factor-kappa B |
| NO | nitric oxide |
| NOX | NADPH oxidase |
| NPPC | natriuretic peptide precursor C |
| PACAP | pituitary adenylate cyclase-activating peptide |
| PAH | pulmonary arterial hypertension |
| PDE | phosphodiesterase |
| PEEP | positive end-expiratory pressure |
| PGD | primary graft dysfunction |
| PIPES | 4-piperazine-bis-ethanesulfonic acid |
| pMDI | pressurized metered-dose inhaler |
| PS | pulmonary sarcoidosis |
| PVR | pulmonary vascular resistance |
| RAMPs | receptor-activity-modifying proteins |
| RCF | receptor component factor |
| RFXP | relaxin family peptide receptors |
| rhDNase | recombinant human deoxyribonuclease I |
| RNS | reactive nitrogen species |
| ROS | reactive oxygen species |
| RVSP | right ventricular systolic pressure |
| SEM | standard error of mean |
| TAPS | [(2-hydroxy-1,1-bis-(hydroxymethyl)ethyl)amino]-1-propanesulfonic acid |
| TAPSE | tricuspid annular plane systolic excursion |
| TBP | TATA-binding protein |
| TES | 2-[tris(hydroxymethyl)methyl]aminoethanesulfonic acid |
| TGF-β | transforming growth factor-beta |
| TNF-alpha | tumor necrosis factor-alpha |
| VEGF | vascular endothelial growth factor |
| VIP | vasoactive intestinal peptide |

## Claims

1. Human anti-inflammatory peptide for use in the prophylaxis or treatment of inflammatory pulmonary diseases by inhalatory administration,
wherein a mesh nebulizer releases for the inhalatory administration an aerosol containing liquid droplets of said human anti-inflammatory peptide according to the invention, and said aerosol is **characterized in that** the fine particle mass is at least 60% of the liquid droplets of the aerosol, and **in that** the median mass aerodynamic diameter of the liquid droplets of the aerosol is between 3.0 µm and 3.5 µm, and
said human anti-inflammatory peptide is selected from a group consisting of vasoactive intestinal peptide, C-type natriuretic peptide, B-type natriuretic peptide, pituitary adenylate cyclase-activating peptide, adrenomedullin, alpha-melanocyte stimulating hormone, relaxin and interferon gamma.

2. The use according to claim 1, wherein said mesh nebulizer is a vibrating mesh nebulizer.

3. The use according to claim 2, wherein said vibrating mesh nebulizer is trigger-activated.

4. The use according to any one of claims 2 or 3, wherein the vibration frequency of the vibrating mesh nebulizer is in the range of 80 kHz to 200 kHz.

5. The use according to any one of claims 1 to 4, wherein the inflammatory pulmonary disease is selected from a group comprising inflammations of the lower airways due to a bacterial, viral, fungal or parasitic infection, chronic lower respiratory diseases, lung diseases due to an external agent, respiratory diseases principally affecting the interstitium, suppurative and/or necrotic conditions of lower respiratory tract, pleura diseases, postprocedural or related lower respiratory diseases, pulmonary diseases specific to the perinatal period, trauma and injuries of the lower respiratory tract and/or the thorax, malignant neoplasms of the lower respiratory tract and inflammatory diseases of the pulmonary circulation.

6. The use according to any one of claims 1 to 5, wherein the inflammatory pulmonary disease is selected from a group comprising chronic obstructive pulmonary disease, asthma, sarcoidosis of the lung, cystic fibrosis, bronchiectasis, adult respiratory distress syndrome, pulmonary fibrosis, berylliosis, chronic lung allograft dysfunction, pulmonary edema, lung ischemia reperfusion injury and primary graft dysfunction after lung transplantation.

7. Human anti-inflammatory peptide as defined in Claim 1 for use in an aerosol for inhalatory administration for the prophylaxis or treatment of inflammatory pulmonary diseases, wherein said aerosol is **characterized in that** the fine particle mass is at least 60 % of the liquid droplets of the aerosol, and **in that** the median mass aerodynamic diameter of the liquid droplets of the aerosol is between 3.0 µm and 3.5 µm.

8. An aerosol produced by a mesh nebulizer, containing
a human anti-inflammatory peptide as defined in Claim 1 in the range of 0.01% per weight to 10 % per weight,
an aqueous solution in the range of 70 % per weight to 99.99 % per weight, and optionally at least one pharmaceutically acceptable excipient in the range of 0 % per weight to 20 % per weight,
wherein said percentages add up to 100 %.

9. A method for producing an aerosol as defined in claim 8, comprising the following steps:
a) filling 0.1 ml to 10 ml of an aqueous solution containing at least one of the human anti-inflammatory peptides as defined in Claim 1 and optionally at least one pharmaceutically acceptable excipient into the nebulization chamber of a mesh nebulizer,
b) starting vibration of the mesh of the mesh nebulizer at a frequency of 80 kHz to 200 kHz, and
c) discharging the generated aerosol at the side of the mesh of the mesh nebulizer opposite to the nebulization chamber.

10. The method according to claim 9,
**characterized in that** at least 50 % in weight of said at least one of the human anti-inflammatory peptides contained in said aqueous solution are nebulized in the generated aerosol.

11. The method according to any one of claims 9 or 10,
**characterized in that** at least 80 % of the generated aerosol are produced during three minutes after starting nebulization in the mesh nebulizer.

12. A kit, comprising a mesh nebulizer and a pharmaceutically acceptable container with an aqueous solution containing at least one of the human anti-inflammatory peptides and optionally at least one pharmaceutically acceptable excipient.

13. A kit, comprising a mesh nebulizer, a first pharmaceutically acceptable container with water for injection or physiological saline solution and a second pharmaceutically acceptable container with a solid form of a human anti-inflammatory peptide as defined in Claim 1,
wherein optionally at least one pharmaceutically acceptable excipient is contained in the first pharmaceutically acceptable container and/or the second pharmaceutically acceptable container.

14. A kit as defined in any one of claims 12 or 13, additionally comprising a mouthpiece for inhalation fitting to said mesh nebulizer.

15. A method of treatment of inflammatory pulmonary diseases, comprising the steps of
a) providing an aerosol as defined in claim 8 by nebulization with a mesh nebulizer, and
b) administering a therapeutically effective amount of said aerosol to a patient in need thereof via a mouthpiece for inhalation fitting to said mesh nebulizer via self-inhalation by the patient.
